Europäisches Patentamt

(19) European Patent Office

Office européen des brevets

(11) **EP 0 667 354 B1**

(12) **FASCICULE DE BREVET EUROPEEN**

(45) Date de publication et mention
de la délivrance du brevet:
**19.09.2001   Bulletin 2001/38**

(51) Int Cl.⁷: **C07K 14/47**, C07K 1/14,
G01N 33/68, C12Q 1/00,
G01N 33/52, G01N 33/60,
A61K 38/17, A61K 39/395

(21) Numéro de dépôt: **95420031.7**

(22) Date de dépôt: **15.02.1995**

(54) **Facteur cytotoxique associé à la sclérose en plaques, sa détection et sa quantification**

Mit Multiple-Sklerose assoziierter, zytotoxischer Faktor, seine Detektion und Quantifizierung

Multiple sclerosis associated cytotoxic factor, its detection and quantification

(84) Etats contractants désignés:
**AT BE CH DE DK ES FR GB GR IE IT LI LU MC NL PT SE**

(30) Priorité: **15.02.1994   FR 9401946**

(43) Date de publication de la demande:
**16.08.1995   Bulletin 1995/33**

(73) Titulaire: **BIO MERIEUX**
**69280 Marcy l'Etoile (FR)**

(72) Inventeurs:
 • **Perron, Hervé**
 **F-38100 Grenoble (FR)**
 • **Dobransky, Tomas**
 **F-78160 Marly-Le-Roy (FR)**
 • **Rieger, François**
 **F-75005 Paris (FR)**
 • **Mandrand, Bernard**
 **F-69100 Villeurbanne (FR)**

(74) Mandataire: **Guerre, Dominique et al**
**Cabinet Germain et Maureau,**
**12, rue Boileau,**
**BP 6153**
**69466 Lyon Cedex 06 (FR)**

(56) Documents cités:
 • **ADVANCES IN EXPERIMENTAL MEDICINE AND BIOLOGY, vol.100, 1978, NEW YORK, N.Y., US pages 19 - 25 A.N. DAVISON ET AL. 'BIOSYNTHESIS OF MYELIN AND NEUROTOXIC FACTORS IN THE SERUM OF MULTIPLE SCLEROSIS PATIENTS.'**
 • **SCIENCE, vol.250, 14 Décembre 1990, LANCASTER, PA US pages 1593 - 1596 D. GIULIAN ET AL. 'SECRETION OF NEUROTOXINS BY MONONUCLEAR PHAGOCYTES INFECTED WITH HIV-1.'**
 • **THE LANCET, vol.337, 6 Avril 1991, LONDON GB pages 862 - 863 H. PERRON ET AL. 'ISOLATION OF RETROVIRUS FROM PATIENTS WITH MULTIPLE SCLEROSIS.'**

**Description**

La présente invention a pour objet un facteur cytotoxique associé à la sclérose en plaques caractérisé par son activité cytotoxique vis à vis des cellules gliales notamment des astrocytes, ainsi que la mise en évidence de cette activité cytotoxique dans un test biologique de détection et de suivi de cette maladie dans des fluides biologiques de patients atteints notamment de sclérose en plaques.

Les cellules gliales (astrocytes, oligodendrocytes, microgliocytes) sont la cible primaire ou secondaire de processus pathologiques dans différentes maladies du système nerveux notamment, chez l'homme, dans les leucoencéphalites, les leucodystrophies, certaines formes d'encéphalopathie, certaines maladies neurodégénératives comme la sclérose latérale amyotrophique où co-existe une gliose astrocytaire avec l'atteinte neuronale et, enfin, des maladies inflammatoires comme la sclérose en plaques ou la maladie de Schilder.

La sclérose en plaques (SEP) est une maladie chronique du système nerveux central de l'homme, évoluant par succession de phases de rémission et de poussée ou selon une progression régulière, et dont la caractéristique anatomopathologique consiste en la formation de zones de démyélinisation bien délimitées dans la substance blanche du cerveau et de la moelle épinière **(1)**. Au niveau histologique, ces zones présentent, au stade précoce du processus lésionnel, une dégradation de la myéline péri-axonale associée à une atteinte des cellules gliales responsables de cette myélinisation, les oligodendrocytes **(2)**. Une activation macrophagique inflammatoire impliquant les cellules microgliales (macrophages tissulaires résidants du système nerveux central) ainsi que, vraisemblablement, des macrophages provenant de monocytes sanguins infiltrés, est associée à ce processus de démyélinisation et contribue à la destruction des feuillets myélinisés **(3)**. Au centre de la zone démyélinisée, une déplétion relative en cellules gliales est retrouvée alors qu'une prolifération d'astrocytes, ou gliose astrocytaire, se développe à la périphérie et peut tardivement envahir la plaque démyélinisée pour générer une plaque fibreuse ou gliotique, telle que l'on en trouve sur le site de lésions anciennes **(1).** Ces structures sclérotiques sont à l'origine du nom donné à la maladie, la "sclérose" en plaques **(4)**.

Une autre caractéristique de ces plaques est leur association quasi systématique avec un élément vasculaire autour duquel elles semblent se développer **(5, 1)**. Au niveau histologique, on y observe une altération fréquente de la barrière hémato-encéphalique (BHE) constituée par l'endothélium capillaire **(6)**. En effet, l'endothélium vasculaire des structures capillaires est normalement jointif dans le système nerveux central, à l'exception des capillaires fenestrés associés aux plexus choroïdes, structures périventriculaires assurant la production du liquide céphalo-rachidien **(7)**. Cette altération de la BHE, marquée par une disjonction des cellules endothéliales et par le passage non régulé d'un flux de liquide plasmatique et de cellules d'origine sanguine dans le parenchyme neuroglial, peut être visualisée au niveau des plaques "actives" par la technique d'imagerie par résonance magnétique (IRM) associée à l'injection intraveineuse, chez le patient examiné, d'une solution de gadolinium. Ce composé permet d'obtenir un signal de résonance magnétique contrasté au niveau du liquide plasmatique et rend détectable le passage anormal de plasma dans le parenchyme nerveux avec l'oedème qui en résulte. On a pu montrer une corrélation entre l'activité lésionnelle des plaques et la présence de cet oedème au même niveau, ainsi qu'entre l'apparition et la résorption de cet oedème et le décours de poussées cliniques de la maladie **(8)**.

Un des éléments déterminants dans le maintien d'une structure jointive de l'endothélium capillaire cérébral, et donc dans celui de la BHE, est constitué par la présence sous-jacente d'extensions cytoplasmiques des astrocytes, appelées pieds astrocytaires **(6)**. Vraisemblablement, ces pieds astrocytaires induisent la formation ou permettent le maintien des structures de jonction étanches (de type zonula occludens) qui assurent la cohésion de la barrière endothéliale capillaire concrétisant la BHE. Or, différents modèles pathologiques font état de l'altération de la BHE associée à une déplétion des pieds astrocytaires **(9, 10)**.

Par ailleurs, dans le processus lésionnel de la SEP, l'altération de la BHE contribue à amplifier la réponse inflammatoire associée, par l'afflux rendu "libre" de cellules lymphoïdes provenant de la circulation sanguine **(11)**.

La contribution de l'inflammation associée aux cellules immunitaires est importante dans la SEP et participe au processus lésionnel, notamment par le biais de lymphocytes et d'anticorps autoréactifs **(12, 13)**. Cependant, contrairement au modèle animal auto-immun de l'encéphalite allergique expérimentale où l'atteinte de la BHE et l'invasion du parenchyme nerveux par des cellules lymphoides du sang circulant initient le processus lésionnel neuroglial **(14)**, on observe dans la SEP que des processus lésionnels précoces associés à une réaction macrophagique locale dans le parenchyme nerveux semblent précéder l'invasion du tissu par les cellules lymphoïdes du sang circulant **(15, 3)**. Il apparait ainsi, que les cellules lymphoïdes, et plus particulièrement les lymphocytes, ne sont pas l'apanage des plaques récentes **(16)**. De plus, la densité de l'infiltration lymphocytaire est surtout marquée dans les zones péri-vasculaires et à la périphérie des plaques de démyélinisation actives **(11, 3)**.

Le stimulus initial à l'origine de la SEP est au coeur du débat sur l'étiologie de la SEP **(17)**. Tour à tour, des arguments ont été mis en avant, en faveur d'une hypothèse virale **(18)**, bactérienne **(19, 20)**, autoimmune **(21, 13)**, toxique **(22)**, ou génétique **(23, 24)**. En fait, il semble qu'une combinaison d'une prédisposition génétique à l'action d'un agent pathogène primaire puisse déboucher sur un processus inflammatoire et auto-immun dévastateur **(25)**.

Différentes séquences d'évènements peuvent ainsi expliquer la démyélinisation et l'atteinte neurologique fonctionnelle dans la SEP, sans qu'il ait été possible à ce jour d'identifier un facteur déterminant qui puisse les initier et donner une explication cohérente à la multitude des données parcellaires accumulées au sujet de cette maladie.

Des molécules d'origine bactérienne ou virale, voire rétrovirale endogène, sont connues pour posséder des propriétés dites superantigéniques **(26, 27)**. Leurs propriétés particulières de stimulation directe des lymphocytes T, spécifiques d'antigènes différents, par liaison avec la région Vβ de certains récepteurs "T", ont fait évoquer l'hypothèse que de telles molécules soient intimement associées au processus étiopathogénique de la SEP **(28)**. Or, un des effets caractéristiques de ces superantigènes sur les cellules T est l'induction prématurée, dans certaines conditions, d'une mort cellulaire programmée ou apoptose **(29)**. Les superantigènes ont aussi pour propriétés de se lier aux molécules HLA de classe II à la surface des cellules présentant l'antigène **(27)**. Rappelons ainsi que les astrocytes possèdent la capacité d'exprimer les antigènes HLA de classe II au niveau de leur membrane plasmique et, notamment, en réponse à certaines cytokines pro-inflammatoires comme l'interféron gamma ou le facteur alpha nécrosant des tumeurs (TNF-alpha) **(30)**. Cependant, aucun superantigène n'a encore été mis en évidence dans la sclérose en plaques.

Par ailleurs, de nombreux processus apoptotiques "intempestifs", par opposition aux processus apoptotiques normaux liés, par exemple, au développement du sytème nerveux **(31)** peuvent intervenir en l'absence de superantigène, qu'il s'agisse d'infection virale **(32)** ou qu'il s'agisse de stimulation d'un récepteur cellulaire **(33)**. Il est intéressant de noter aussi qu'une apoptose peut être induite par la stimulation in vitro d'un récepteur membranaire au TNF-alpha **(31)**, mais qu'un tel phénomène n'a pas été étudié dans le système nerveux et a fortiori avec les astrocytes.

Certaines cytokines peuvent donc déclencher un processus pathogène et être produites, notamment par les macrophages, et ont un effet cytotoxique sur les oligodendrocytes **(34)**. Il est à noter que le TNF alpha, ainsi que l'interleukine-1-alpha, -1-alpha, l'interleukine-1-béta, l'interleukine-2, l'interleukine-6 et l'interféron gamma, n'ont pas a priori d'effet cytolytique sur les astrocytes, mais induisent plutôt une prolifération astrocytaire **(35)**. Cependant, les astrocytes peuvent eux-mêmes être soumis à des stimulations à l'origine d'une sécrétion de TNF alpha. Ceci peut s'observer, par exemple, en réponse à des toxines bactériennes lipopolysaccharidiques ou à des ionophores calciques **(36)**. Ainsi, une atteinte des oligodendrocytes, et donc une destruction des feuillets myéliniques, peut se produire indirectement par le biais du TNF alpha produit par des astrocytes. Toute molécule induisant une telle production de cytokine par les astrocytes, quel qu'en soit l'effet propre sur ces derniers (prolifération, différenciation ou effet cytopathogène), est donc potentiellement démyélinisante. Le TNF-alpha induit un mécanisme cytotoxique oligodendrocytaire dont les premiers effets sont marqués par un gonflement des structures myéliniques, évoquant une action médiée par les canaux ioniques **(34)**. Une corrélation entre la production de TNF-alpha in vivo et les poussées de SEP semble d'ailleurs exister **(37)**. Le rôle des astrocytes dans une telle production de TNF-alpha chez les patients atteints de SEP est cependant méconnu.

En tout état de cause, les astrocytes étant susceptibles de produire du TNF-alpha et de co-présenter un antigène-cible avec des antigènes HLA de classe II aux cellules immunocompétentes, elles-mêmes recrutées par un processus cytopathique et/ou inflammatoire, ils se trouvent de fait au carrefour des interactions immunopathologiques telles qu'on peut en observer dans le processus lésionnel démyélinisant qui caractérise la SEP.

Un autre type de molécule susceptible de jouer un rôle dans le processus pathogénique conduisant à la formation d'une plaque de démyélinisation, puis à une gliose astrocytaire, est constitué par les protéines dites de choc thermique (HSP) ou protéines de stress. Ces protéines constituent une famille phylogénétique relativement conservée et sont retrouvées tant chez les procaryotes que chez les vertébrés supérieurs **(38)**. Leur synthèse est inductible dans les cellules eucaryotes par différents stress, notamment infectieux ou thermiques, et leur communauté antigénique interespèces a fait évoquer une induction possible d'autoimmunité chez l'homme par des bactéries infectieuses portant des HSP, comme mycobacterium tuberculosis **(39)**.

Des antigènes bactériens pouvant posséder des propriétés superantigèniques ou être des protéines de choc thermique, ont été incriminés dans l'induction de poussées de SEP, sans qu'il soit clairement établi à ce jour s'ils s'agit de co-facteurs de pathogénicité occasionnels ou d'agents étiologiques, ou encore de pathogènes partageant des propriétés communes responsables d'une pathogénicité identique **(40, 41)**.

Ce type de mécanisme fait intervenir, d'une part la présence d'HSP exogènes (bactériennes, par exemple) contre lesquelles les cellules immunocompétentes se sensibilisent, et d'autre part, l'expression d'HSP endogènes (de l'organisme infecté) à la surface de cellules exprimant les antigènes HLA de classe II. Si les HSP cellulaires partagent des épitopes communs avec les HSP exogènes, elles peuvent être reconnues comme "infectieuses" par les lymphocytes sensibilisés. Leur rôle a aussi été évoqué dans la SEP **(42)**. La "gliotoxicité" de telles molécules peut passer par la réponse immunitaire et, éventuellement, par les cellules astrocytaires ou les microgliocytes (autres cellules gliales macrophagiques pouvant présenter un antigène spécifique), mais les effets cytopathiques propres de certaines HSP sur les cellules gliales sont méconnus au vu de l'absence d'études exhaustives dans ce domaine.

Des travaux effectués ont apportés des arguments en faveur d'une étiologie virale de la SEP **(notamment 43, 44, 45 et WO-93/20188 dont le contenu est incorporé à titre de référence).**

A la suite des travaux précités, les présents inventeurs ont été conduits à rechercher un ou des facteurs effecteurs du processus pathogénique aboutissant à la formation typique de plaques de démyélinisation et à une gliose astrocytaire.

Bien que les cultures de monocytes / macrophages sanguins de sclérose en plaques aient contribué à étayer une hypothèse virale au cours des travaux précités, d'autres aspects du rôle des cellules macrophagiques dans la pathogénie de cette maladie, qui n'impliquent pas nécessairement un agent viral, doivent être pris en considération ; à cet égard, une hypothèse a donc été émise et vérifiée selon laquelle un ou plusieurs facteur(s) toxique(s) pour les cellules macrogliales (astrocytes, oligodendrocytes) pourrait(ent) être produit(s) par les monocytes de patients atteints de SEP.

Bien que les facteurs moléculaires en cause soient méconnus, un facteur toxique provenant soit des cellules immunitaires ou inflammatoires, soit d'un agent viral ou bactérien, soit encore, induit par ces derniers dans les cellules environnantes, est susceptible d'initier un processus aboutissant à une réponse inflammatoire aigüe ou chronique.

La double interrogation sur l'origine rétrovirale éventuelle de la SEP **(46)** et sur la contribution éventuelle d'un agent bactérien à sa pathogénie **(40, 47)** conforte les inventeurs dans leur recherche d'éléments cytotoxiques pour les cellules macrogliables chez les malades atteints de SEP. En effet, ce sont les cellules gliales qui constituent la cible principale du processus neuropathologique dans la SEP.

A.N. DAVISON et coll **(48)** ont étudié l'activité des oligodendrocytes, qui sont les cellules impliquées dans la synthèse de la myéline, et notamment les facteurs susceptibles d'intervenir comme inhibiteur de la synthèse de la myéline. Ces travaux sont cependant exclusivement limités aux oligodendrocytes, et ne permettent pas d'avancer dans l'explication du processus pathogénique décrit ci-dessus.

L'invention a ainsi pour objet un facteur gliotoxique, à l'état isolé ou purifié, qui possède une activité toxique vis-à-vis des cellules astrocytaires humaines ou animales, ladite activité ayant pour effet une désorganisation cytomorphologique de leur réseau de filaments intermédiaires et/ou une dégradation des protéines desdits filaments intermédiaires et/ou une mort cellulaire notamment par apoptose.

Par facteur gliotoxique, on entend, une molécule particulière, ou un facteur représenté par un ensemble de molécules pouvant être définies, présentant une activité biologique pouvant être mise en évidence par un effet cytotoxique sur des cellules gliales.

Les inventeurs ont pu mettre en évidence que l'activité toxique du facteur précité était associé à au moins une glycoprotéine globulaire.

Selon l'invention, on entend par glycoprotéine, une protéine à laquelle est associée par liaison covalente, au moins un groupement glucidique.

Ils ont en outre caractérisé un facteur gliotoxique qui s'avère selon l'invention, après traitement <u>d'un échantillon biologique le contenant,</u> successivement, sur une résine échangeuse d'ions puis sur une colonne de séparation par exclusion, constitué, majoritairement, par une fraction légère, centrée sur un poids moléculaire apparent d'environ 17 KD, et minoritairement, par une fraction lourde, centrée sur un poids moléculaire apparent d'environ 21 KD, au moins ladite fraction légère étant résistante, dans des conditions non dénaturantes, à l'action hydrolytique de la pronase, de la trypsine ou de la protéinase K, et chacune des deux dites fractions présentant une forte affinité pour les lectines et notamment la concanavaline A.

Le facteur gliotoxique selon l'invention présente une utilité majeure dans le diagnostic, mais aussi dans la prophylaxie et la thérapeutique de la SEP.

Selon l'invention, un facteur gliotoxique est susceptible d'être obtenu à partir du procédé comprenant les étapes suivantes:

- on part d'un échantillon biologique prélevé par exemple sur un patient atteint de sclérose en plaques cliniquement active,
- on traite successivement ledit échantillon sur une résine échangeuse d'ions puis sur une colonne de séparation par exclusion, pour obtenir un facteur gliotoxique constitué majoritairement, par une fraction légère, centrée sur un poids moléculaire apparent d'environ 17 KD, et minoritairement, une fraction lourde, centrée sur un poids moléculaire apparent d'environ 21 KD, chacune desdites fractions possédant une activité gliotoxique et ayant en outre les propriétés de résistance et/ou d'affinité mentionnées ci-dessus.

Par échantillon biologique selon l'invention, on comprend notamment un prélèvement, du type fluide, tissu ou fragment de tissu, mucosité, organe ou fragment d'organe, ou surnageant de cultures obtenu à l'aide d'un des prélèvements précités.

Un autre objet de l'invention est un procédé pour détecter et/ou suivre l'activité et/ou pronostiquer une pathologie telle que la sclérose en plaques, consistant à détecter, dans un échantillon biologique, la présence et/ou la quantité d'un facteur gliotoxique tel que défini par l'invention.

De préférence, l'échantillon biologique contenant le facteur gliotoxique subit un procédé de prétraitement, caractérisé en ce que, pour éliminer des contaminants susceptibles de produire une activité cytotoxique parasite et non spécifique du facteur gliotoxique de l'invention, on effectue un traitement dudit échantillon avec l'un au moins des traitements suivants:

- on met ledit échantillon en contact avec de la protéine A,
- on met ledit échantillon en contact avec une résine échangeuse d'ions,
- on met ledit échantillon en contact avec une lectine et notamment la concanavaline-A.

La présente invention concerne aussi un procédé pour détecter et/ou quantifier, dans un échantillon biologique, l'activité toxique du facteur gliotoxique décrit précédemment consistant à incuber ledit échantillon biologique, dans un milieu de culture approprié et contenant des astrocytes notamment immortalisés, permettant leur culture, et à détecter et/ou quantifier les astrocytes morts et/ou les astrocytes vivants.

Pour détecter et/ou quantifier les astrocytes morts et/ou les astrocytes vivants, on peut mettre en oeuvre différentes techniques de dosage et en particulier, les suivantes :

*    un dosage colorimétrique mettant en oeuvre respectivement la calcéine-AM et l'éthidium homodimère
*    un dosage colorimétrique mettant en oeuvre le bromure de méthyltétrazolium
*    un dosage radioactif mettant en oeuvre le $^{51}$Cr.

Selon l'invention, un autre procédé pour détecter et/ou quantifier, dans un échantillon biologique, l'activité toxique d'un facteur gliotoxique comprend une étape d'incubation dudit échantillon dans un milieu de culture approprié, contenant des astrocytes, notamment immortalisés, permettant leur culture, et une étape de détection et/ou de quantification de la fragmentation de l'ADN des astrocytes, et/ou de la désorganisation cytomorphologique du réseau des filaments intermédiaires et/ou de la dégradation des protéines desdits filaments intermédiaires.

Dans un procédé de l'invention pour détecter, dans un échantillon biologique, la présence d'un facteur gliotoxique, on utilise les propriétés de ce dernier en réalisant une étape de capture dudit facteur par une lectine et notamment la concanavaline-A, et/ou une étape de détection basée sur l'affinité dudit facteur pour unedite lectine.

Enfin, un dernier objet de l'invention est une composition diagnostique, et/ou thérapeutique, et/ou prophylactique, comprenant un facteur gliotoxique de l'invention.

Plus généralement, la présente invention peut être appliquée à un dispositif pour mettre en oeuvre un test biologique utilisant par exemple une technique ELISA, possédant un support, ou un substrat, susceptible de se lier au facteur de l'invention.

Dans tout le texte, les exemples, tableaux et figures annexées, quand le diagnostic de SEP est mentionné, il s'entend comme un diagnostic de SEP certaine définie par les critères de Poser **(49)**.

La présente invention sera mieux comprise à la lecture de la description détaillée qui va suivre, faite en référence aux figures annexées dans lesquelles:

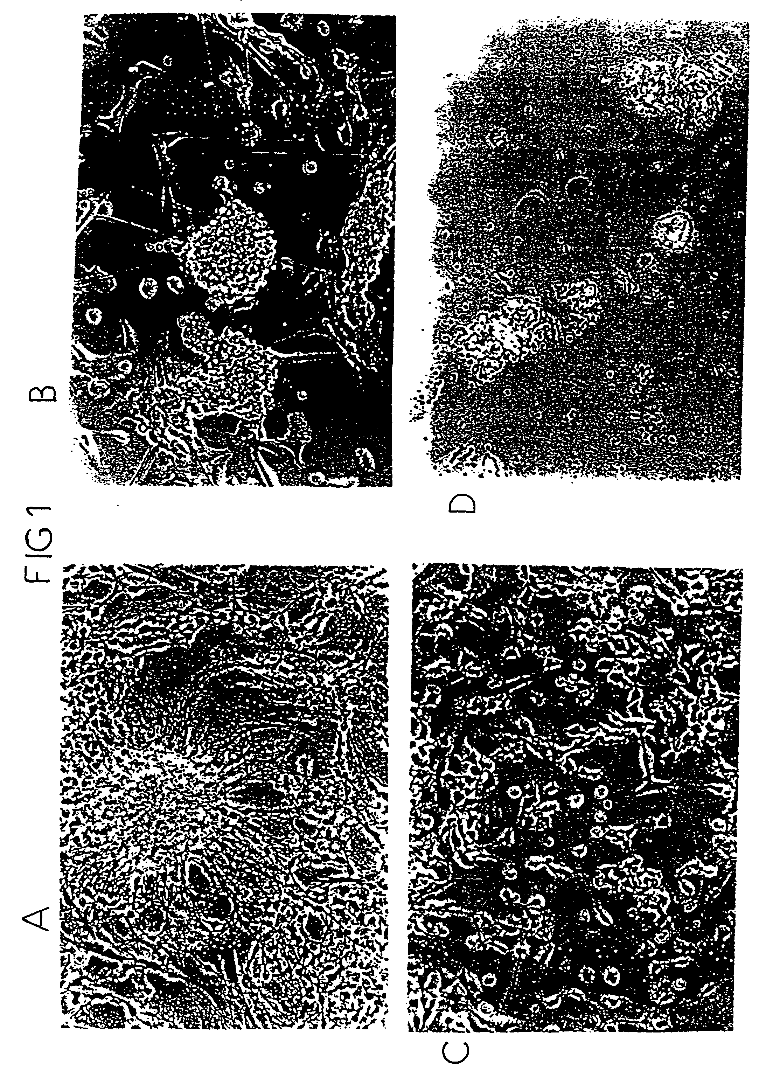

La figure 1 illustre l'effet cytotoxique, observé au microscope optique à contrast de phase, dans des cultures d'explants de cerveau embryonnaire de rat.

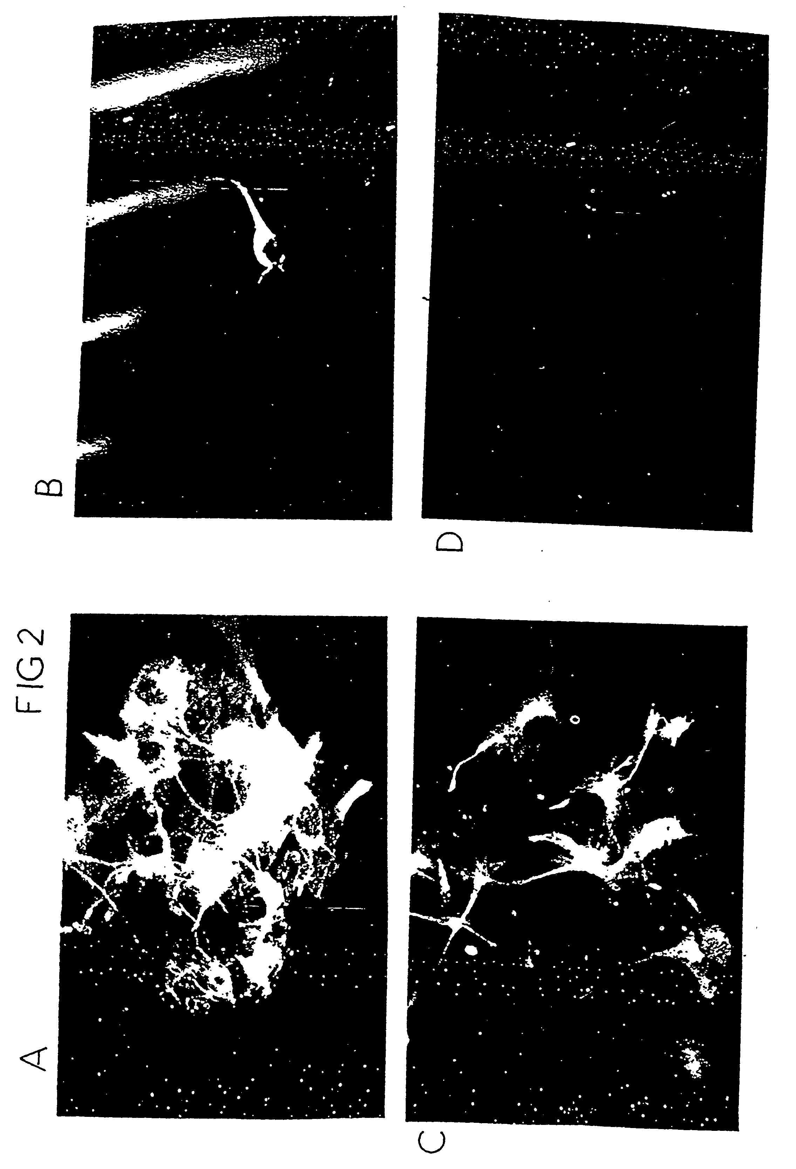

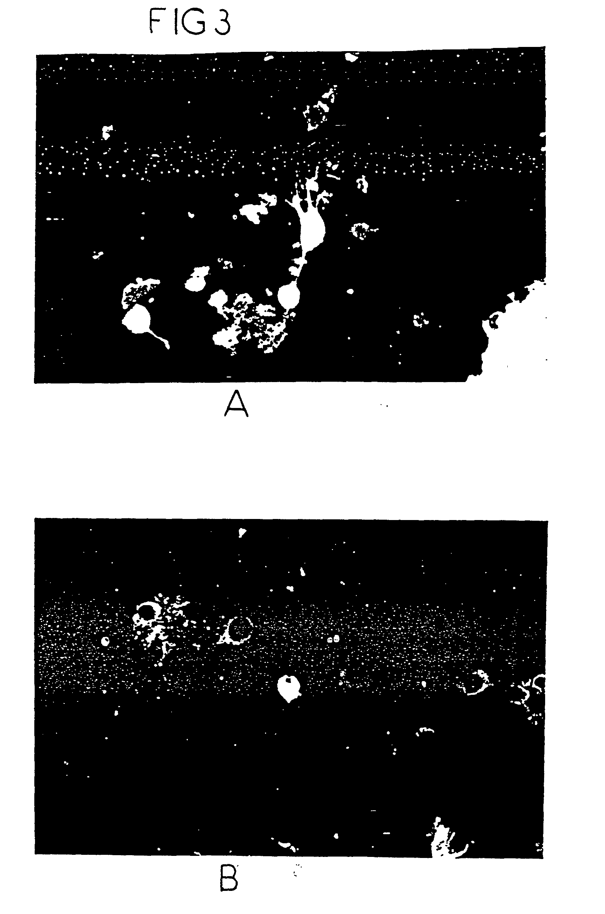

Les figures 2 et 3 montrent l'effet cytotoxique observé sur différent types de cellules présents dans les cultures d'explants embryonnaires, par analyse immunocytologique au microscope à fluorescence à une longueur d'onde appropriée pour la détection d'un signal émis par un anticorps secondaire utilisé dans une procédure d'immunofluorescence indirecte et marqué à la fluorescéine.

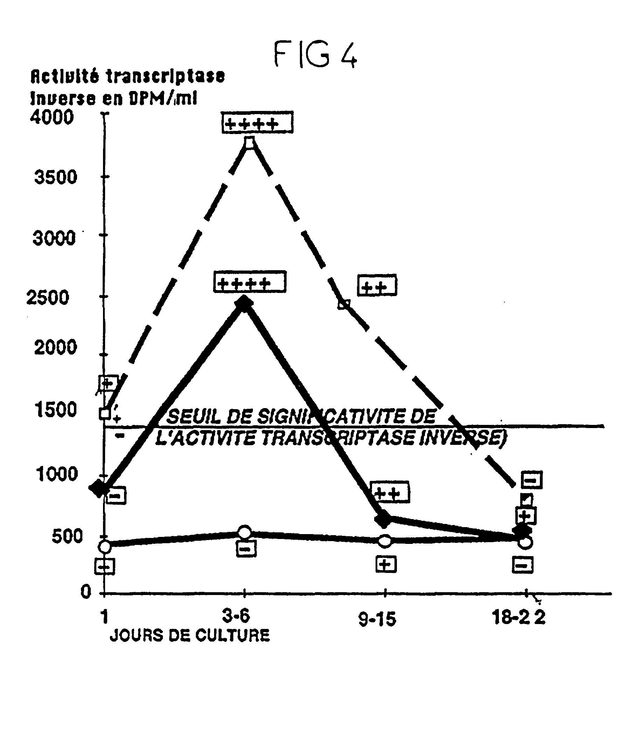

La figure 4 illustre la coincidence, dans quelques cultures de monocytes/macrophages de sclérose en plaques, entre les activités transcriptase inverse et cytotoxique. L'activité transcriptase inverse est donnée en dpm (désintégrations par minute) en fonction du nombre de jours de culture. La courbe en pointillés représente une culture de monocytes/macrophages d'un patient présentant une forme chronique fortement progressive de SEP, la courbe continue avec des losanges noirs représente une culture de monocytes/macrophages d'un patient présentant une forme rémittante de SEP en poussée aigüe et la courbe continue avec des ronds blancs représente une culture de monocytes/macrophages d'un patient présentant une forme rémittante de SEP en période de rémission. L'activité cytotoxique est représentée par un nombre de croix allant de un à quatre (effet cytotoxique positif allant de très faible à très fort) ou un signe négatif (absence de cytotoxicité), en regard du point porté sur la courbe représentant l'activité transcriptase inverse du surnageant correspondant.

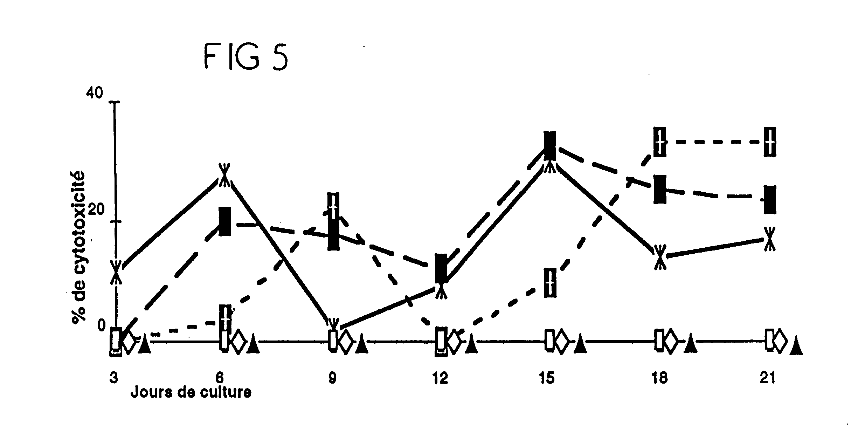

La figure 5 illustre en fonction des jours de culture la cinétique de l'expression de l'activité cytotoxique à partir de surnageants de cultures provenant de patients atteints de SEP et de témoins. Sur le graphique, les trois courbes en trait plein ou pointillé représentent les cinétiques de gliotoxicité dans les surnageants de culture de monocytes/macrophages de trois cas de SEP différents et les points alignés sur l'axe des abcisses (triangle noir, losange et rectangle blancs) les cinétiques négatives de culture de monocytes/macrophages de trois témoins atteints d'autres maladies neurologiques.

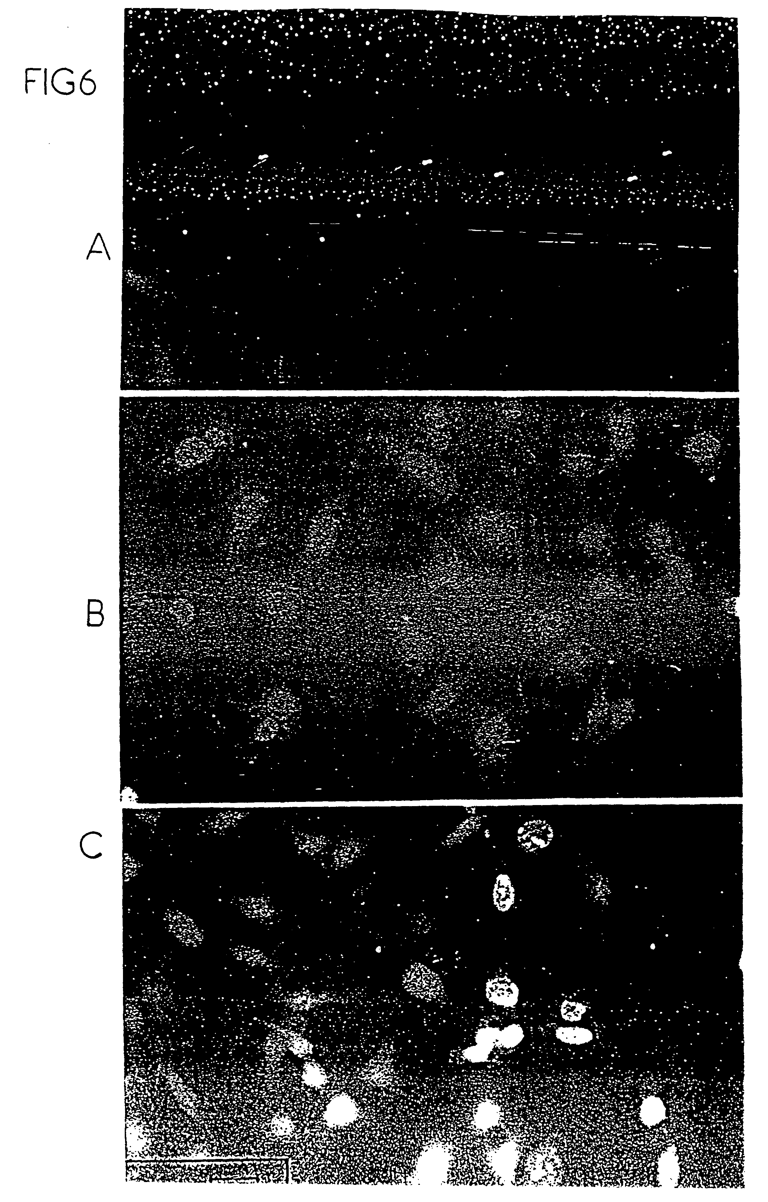

La figure 6 représente une visualisation de cellules astrocytaires vivantes et/ou mortes avec un système optique.

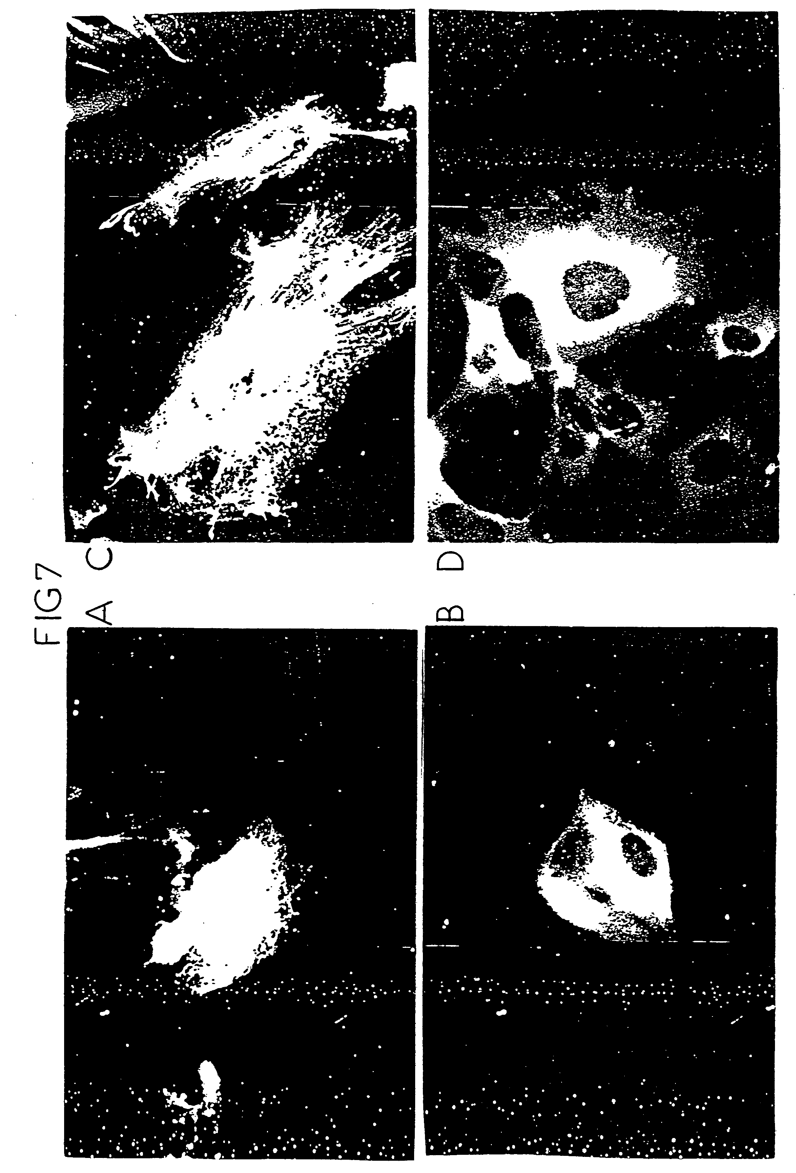

La figure 7 montre les effets cytologiques liés à la cytotoxicité sur les astrocytes.

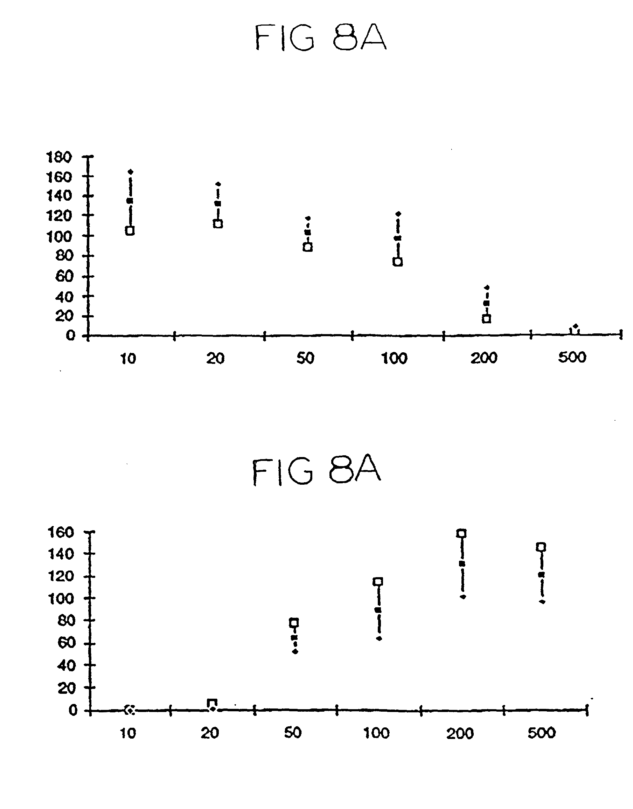

La figure 8 illustre le nombre moyen de cellules mortes (Fig 8A) ou vivantes (Fig 8B) astrocytaires après mélange à un surnageant de culture monocytes/macrophages à différentes dilutions. Les moyennes des comptages des cellules mortes et vivantes en fonction de la dilution de l'échantillon gliotoxique sont représentées par des carrés noirs sur deux graphiques séparés. Les intervalles de confiance correspondant à chaque moyenne sont représentés par une valeur supérieure et une valeur inférieure distantes de deux écart-types.

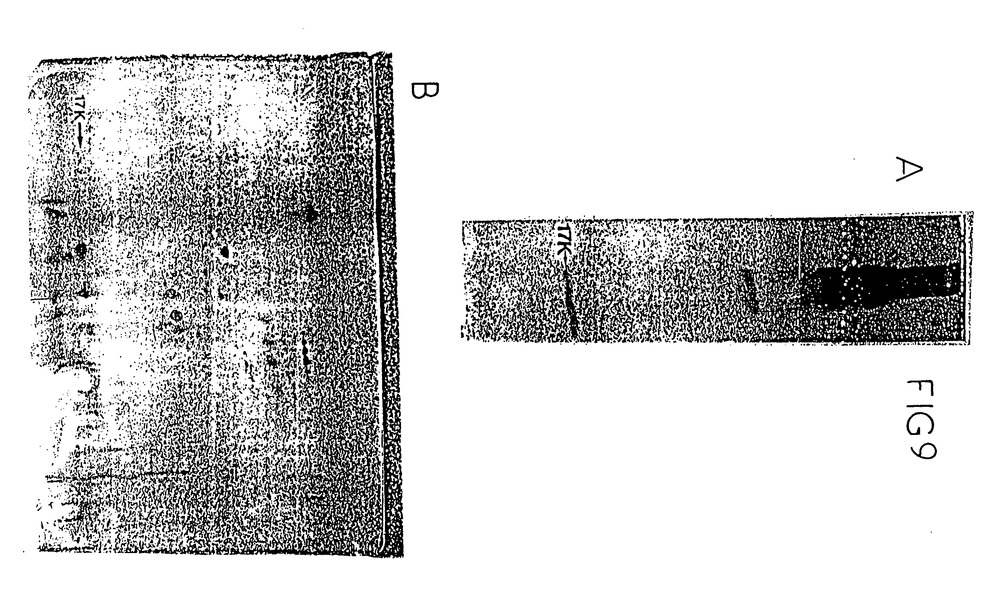

Les figures 9A et 9B représentent l'électrophorèse sur gel d'acrylamide respectivement en une puis deux dimensions d'une fraction gliotoxique obtenue à partir de filtres utilisés pour la filtration in vivo de liquide céphalorachidien (LCR) de patients atteints de sclérose en plaques. Préalablement à l'électrophorèse les molécules adsorbées sur le filtre ont été éluées puis passées sur une résine échangeuse d'ions.

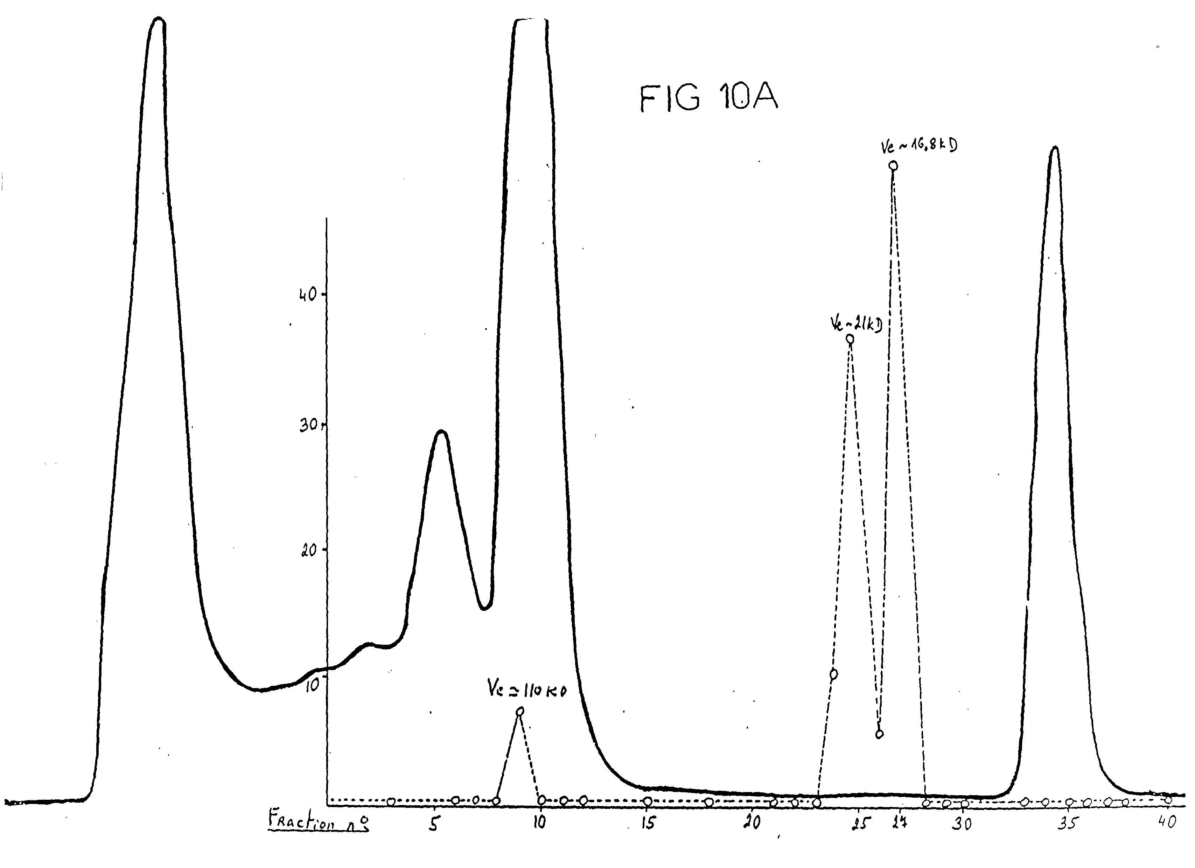

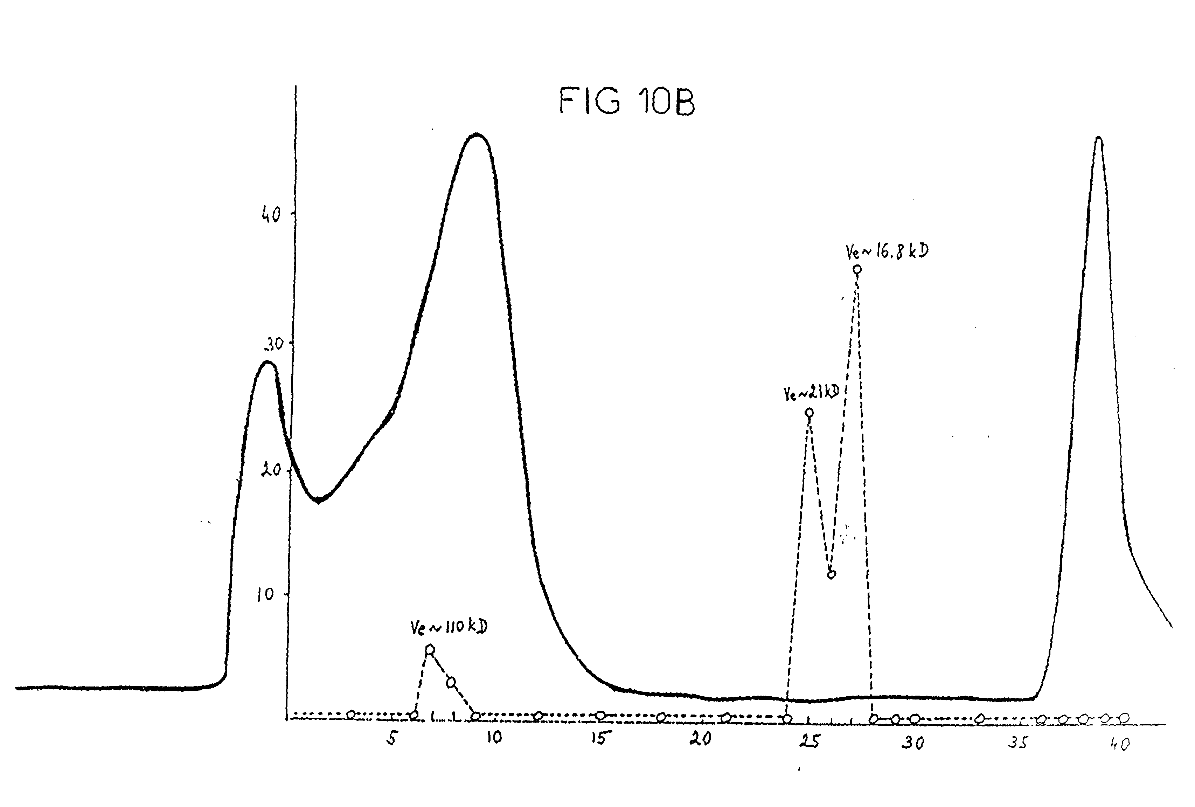

Les figures 10A et 10B représentent l'élution dans un tampon Tris-HCl 50 mM, pH 6,8 sur colonne d'exclusion superose 12 d'une fraction gliotoxique obtenue après passage sur résine DEAE Sépharose et provenant d'un mélange de surnageants de culture de monocytes/macrophages de SEP prélevés à $J_6$, $J_9$, $J_{12}$, $J_{16}$ selon Figure 10A et prélevés à $J_6$, $J_9$, $J_{13}$, $J_{16}$ selon Figure 10B ; le trait épais représente l'absorption des composants protéiques à 280 nm avec un coefficient de sensibilité R = 1,2 ; le trait en pointillé représente l'activité gliotoxique mesurée sur des fractions successives selon le protocole décrit plus loin dans la description. Les poids moléculaires apparents correspondant aux volumes d'élution (Ve) des pics de gliotoxicité sont indiqués au sommet de ces pics.

Selon la figure 10A, l'élution est faite en l'absence d'urée et selon figure 10B, l'élution est faite en présence d'urée 8M.

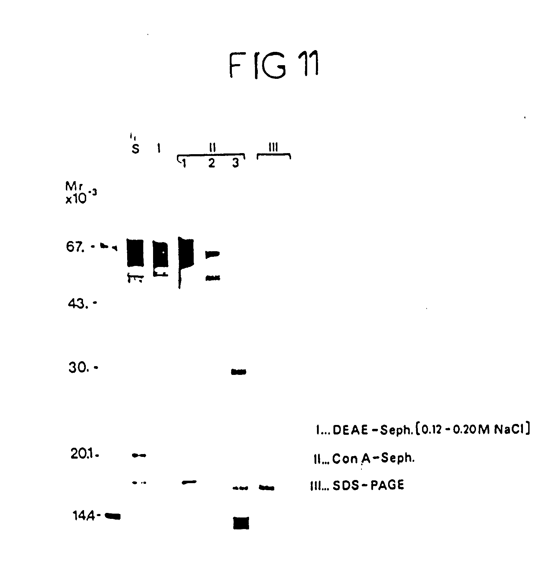

Dans la figure 11, est présenté un gel à une dimension, d'électrophorèse SDS-polyacrylamide, montrant dans les puits successifs, la réalisation d'une purification protéique du facteur gliotoxique partant d'un surnageant brut de cultures de monocytes/macrophages de SEP et aboutissant après passage sur colonne DEAE puis concanavaline-A à l'isolement de bandes protéiques de 17 et 21 KD portant la quasi-totalité de l'activité de l'échantillon.

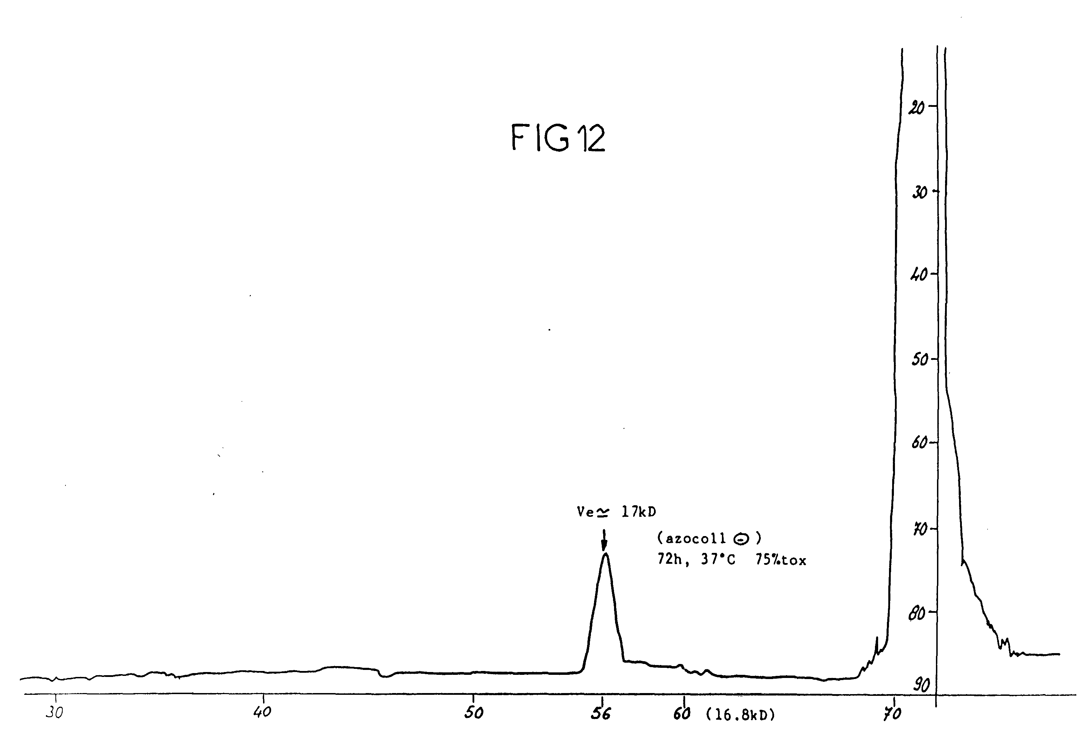

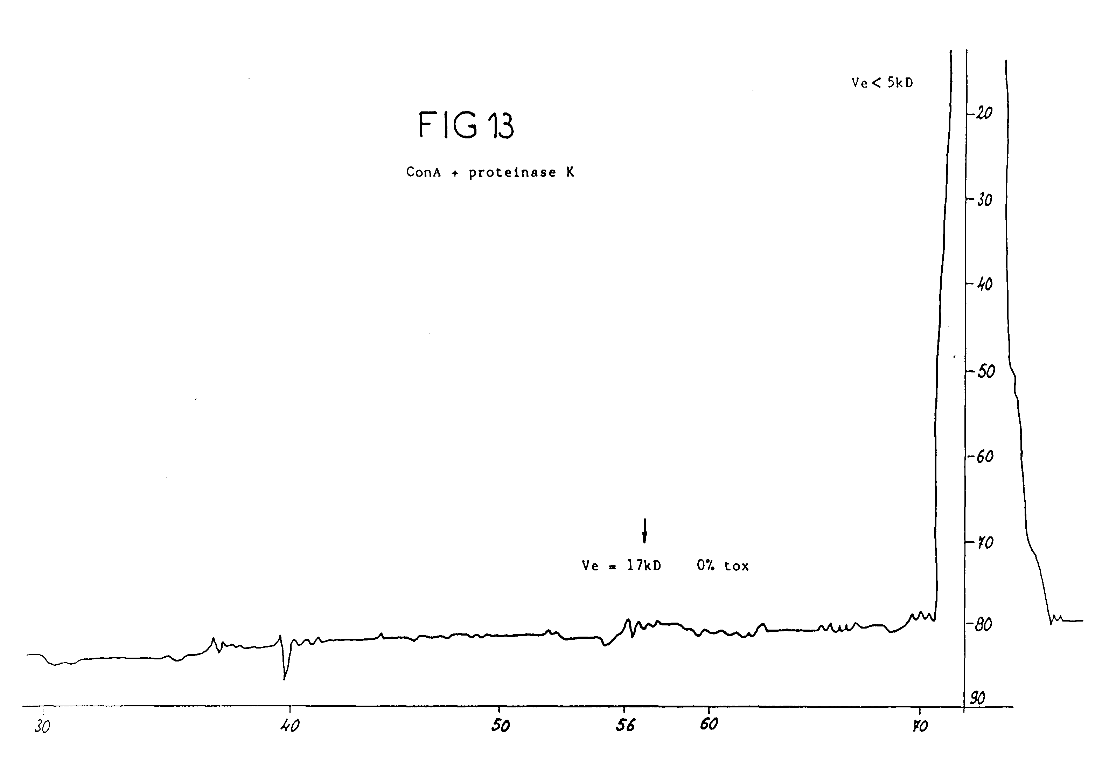

Dans les figures 12 et 13 sont présentées les élutions, sur colonne superose 12 dans un tampon contenant 50 mM Tris-HCl, pH 6,8, avec de l'urée 8M, de fractions préalablement obtenues dans l'éluat en tampon glycine pH 3 de colonne Con-A-Sépharose. Ces éluats ont été incubés en présence de protéinase K avant passage sur superose 12.

La figure 12 représente une fraction issue d'un surnageant gliotoxique de culture ou monocytes de SEP et la figure 13 représente une fraction issue d'un surnageant de culture témoin non gliotoxique.

La courbe représente à chaque fois l'absorption à 280 nm des composants peptidiques avec une sensibilité de détection R = 0,02.

Le niveau d'élution de protéine standard de 17 KD est indiqué par une flèche.

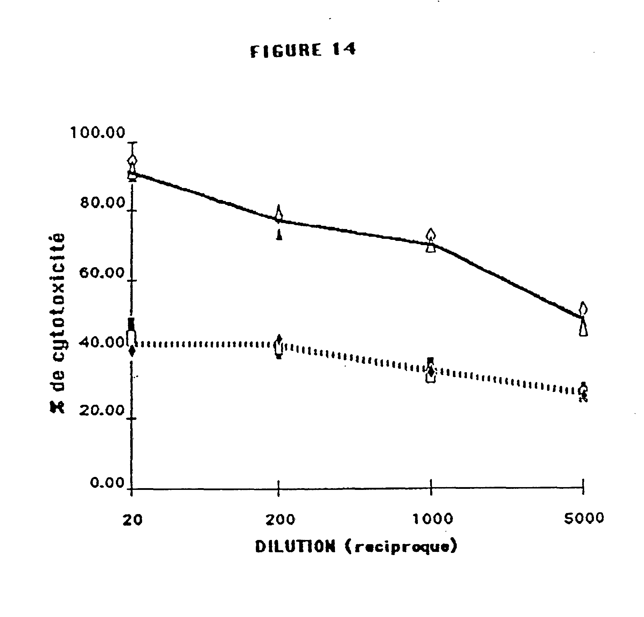

La figure 14 représente l'effet dose-réponse dans un test de cytotoxicité au $^{51}$Cr après 72 h d'incubation, la courbe en pointillé correspondant aux valeurs obtenues pour un surnageant de culture de monocytes de SEP, et la courbe en trait plein correspondant aux valeurs obtenues pour une fraction purifiée sur Con-A provenant du même surnageant de culture.

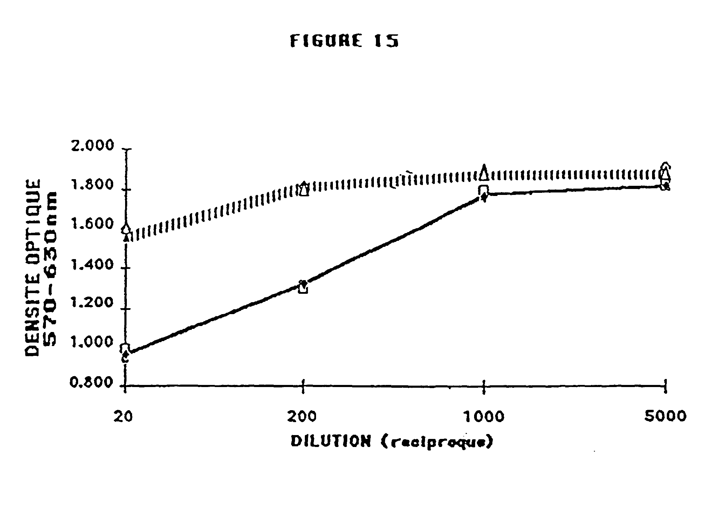

La figure 15 représente l'effet dose-réponse dans un test de cytotoxicité au méthyltétrazolium, après 72 h d'incubation, la courbe en pointillé correspondant aux valeurs obtenues, en trois mesures, pour un surnageant de culture de monocytes de SEP, et la courbe en trait plein correspondant aux valeurs obtenues pour une fraction purifiée sur Con-A provenant du même surnageant de culture.

Les premiers travaux ont utilisé comme fluides biologiques à analyser, les surnageants de culture in vitro de monocytes/macrophages de patients atteints de SEP, de témoins sains ou atteints d'autres maladies neurologiques, et, comme support de détection d'un effet cytotoxique, des cultures in vitro d'explants de cortex cérébral embryonnaire de rat.

## EXEMPLE 1: Culture de monocytes/macrophages sanguins

Le milieu de culture comprend du RPMI1640 (Boehringer), de la pénicilline-streptomycine (bioMérieux), de la L-glutamine (bioMérieux), du pyruvate de sodium (Boehringer), des acides aminés non-essentiels 100x (Boehringer), du sérum humain AB prélevé chez des donneurs sains et séronégatifs pour tous les virus transmissibles par les dérivés sanguins connus.

Les cellules lymphoïdes sont cultivées dans des flacons de culture de 75 cm$^2$ Primaria (Falcon) après avoir été séparées du plasma et des autres éléments figurés sanguins par centrifugation sur gradient de Ficoll (Lymphoprep® , Flow). Pour obtenir ces cellules lymphoïdes, 50 ml de sang sont prélevés par ponction veineuse sur tube stérile hépariné (lithium héparine). Le sang et l'héparine sont bien mélangés aussitôt le sang prélevé. Alternativement, le sang peut être prélevé dans des tubes contenant de l'EDTA. Il est important ensuite, de transporter immédiatement les tubes maintenus à +4°C au laboratoire, où ceux-ci seront manipulés sous hotte de culture à flux laminaire "biohazard" dans des conditions stériles. Par prélèvement, on prépare un milieu "RPMI" qui pourra avantageusement comprendre 100-150 ml de milieu RPMI 1640, un mélange de pénicilline et streptomycine, 4% de L-glutamine, 1% de pyruvate de sodium, 1% d'acides aminés non-essentiels Boehringer (100X), ainsi que 3 tubes stériles à fond conique de 50 ml (Falcon) contenant 10 ml du milieu "RPMI" sus-décrit, et 4 tubes stériles de 50 ml avec 20 ml de Ficoll au fond. Les tubes héparinés sont ouverts pour pipeter le sang, le déposer dans les tubes contenant du milieu et le mélanger doucement au milieu sus-décrit. On prélève 5 ml de milieu "RPMI" et l'on rince la paroi des tubes héparinés. Il faut accompagner ce rinçage d'un grattage léger à l'aide du bout de la pipette plastique afin de décoller des cellules ayant éventuellement adhéré aux parois du tube, et le déposer dans les tubes contenant le sang dilué dans le milieu "RPMI", en mélangeant doucement le contenu par aspirations/refoulements successifs. Il faut répéter ces opérations jusqu'à ce que les tubes héparinés soient propres. Il faut ensuite déposer très doucement (sans remous) le sang dilué dans le milieu "RPMI", à la surface du Ficoll dans les tubes de 50 ml puis utiliser du milieu "RPMI" pour rincer le reste de sang dilué et le récupèrer comme précédemment pour le déposer délicatement à la surface des tubes avec le Ficoll. Ensuite, et sans secouer le Ficoll, il faut placer les tubes dans des godets pour centrifugeuse, équilibrer à l'aide de tubes remplis d'eau, et centrifuger à +15°C pendant 20 minutes à 1800 tr/min, avec un mode de décélération lente. Après centrifugation, on récupère les tubes dans lesquels on enfonce doucement une pipette jusqu'à hauteur de l'interface supérieure "Ficoll/plasma" et l'on aspire doucement la couche blanchâtre située au dessus du Ficoll en décrivant des cercles concentriques depuis les parois, puis en décrivant des "zig-zags" d'un côté à l'autre de la surface du Ficoll. On place le milieu aspiré dans des tubes de 50 ml, on le dilue dans au moins 3 fois le volume de milieu RPMI et l'on mélange doucement par inversion des tubes bouchés stérilement. On centrifuge ensuite les tubes à +15°C pendant 10 minutes à 1800 tr/min, avec un mode de décélération lente. Après centrifugation, on jette en versant lentement mais régulièrement le surnageant de ces tubes, tout en en veillant à ce que le culot blanchâtre de cellules ne se détâche pas. On resuspend le culot dans 10 ml de milieu "RPMI" par aspirations/refoulements successifs et on centrifuge la suspension à +15°C pendant 10 minutes à 1800 tr/min, avec un mode de décélération lente. Par prélèvement ou par 50 ml de sang prélevé, on prépare deux petits flacons de culture en plastique electropositif (Falcon "PRIMARIA") de 75 cm$^2$ et 10 ml de milieu "RPMI" auquel on aura ajouté 15% de sérum humain "AB" (SH) sus-décrit. Après centrifugation, on élimine le surnageant comme précédemment, on resuspend doucement le culot dans 5 ml de milieu "RPMI" avec 15% de SH et l'on répartit les cellules resuspendues dans les flacons placés à plat et à peine soulevés. La suspension est aussitôt répartie en remuant chaque flacon à plat. Les tubes de centrifugation sont rincés avec 5 ml de milieu "RPMI" à 15% SH, et la suspension est ajoutée et répartie dans les deux flacons, comme précédemment. Avantageusement, tous les milieux utilisés pour ces étapes sont à 37°C (réchauffés au bain marie). Une fois les flacons refermés, ils sont tenus à plat dans une étuve humide à 37°C avec 5% de $CO_2$ jusqu'au lendemain matin. Le lendemain matin, il convient de bien aspirer tout le surnageant avec les cellules en suspension, de rincer les flacons deux fois avec 4 ml de RPMI seul, en laissant "tremper" 5 minutes à chaque fois et en relevant lentement le flacon avant d'aspirer tout le milieu restant, afin d'éliminer les cellules non-adhérentes. On remplit ensuite les flacons de 5 ml de milieu RPMI à 15% de SH, on les replace à l'étuve et l'on veille à ne pas les bouger pendant 4 h. Dès cette étape, il convient de toujours remplir les flacons places debout en dirigeant le jet sur la paroi superieure afin de ne pas détacher les cellules en cours d'adhésion, puis, par la suite, affectées par un effet cytopathogène éventuel. Les suspensions cellulaires ainsi recueillies 24 h après la mise en culture, sont centrifugées à +15° C pendant 10 minutes à 1800 tr/min, avec un mode de décélération lente. Eventuellement, le culot de cellules peut être repris dans du sérum de veau foetal avec 10% de DMSO (Diméthyl sulfoxide) pour être congelé à -80°C ou dans l'azote liquide selon une procédure de maintien de cellules viables. Le surnageant correspondant est ensuite centrifugé à 3000 tr/min pendant 30 min. afin d'éliminer les débris cellulaires, et le surnageant clarifié est aliquoté, répertorié comme échantillon à 24 h de culture, soit J1, puis stocké au congélateur à -80°C. Après 48 h dans l'étuve, on sort les flacons, le surnageant est aspiré délicatement, et, comme précédemment centrifugé à 3000 tr/min pendant 30 minutes afin d'éliminer les débris cellulaires. Le surnageant clarifié est aliquoté, répertorié comme échantillon à 3 jours de culture, soit J3, puis stocké au congélateur à -80°C. Les flacons sont aussitôt remplis avec 5 ml de milieu RPMI à 5% SH et replacés dans l'étuve. A partir de ce moment, le milieu de culture ne contient plus que 5% de SH et cette proportion sera utilisée pour tous les renouvellements de milieu. Les milieux des flacons sont ensuite prélevés, stockés sous aliquots de milieu clarifié des débris cellulaires, à -80°C comme précédemment, et remplacés par du milieu "RPMI" à 5% de SH, tous les trois ou quatre jours, jusqu'à ce que ne persiste plus aucune cellule adhérente et réfringente à l'observation microscopique dans le flacon.

**EXEMPLE 2: Test de cytotoxicité sur cultures d'explants de cortex cérébral embryonnaire de rat et d'explants de moelle épinière embryonnaire de rat.**

Les cultures d'explants de cortex cérébral embryonnaire de rat, sont obtenues à partir de cerveaux d'embryons de rats prélevés sur ratte gestante, au quatorzième jour de vie embryonnaire. Après dissection, les cerveaux sont rincés trois fois dans un tampon "Dulbecco-Phosphate Buffer Saline (D-PBS: $KH_2PO_4$ 0,2 g/l, $Na_2HPO_4$-$7H_2O$ 1,15 g/l, NaCl 8 g/l, KCI 0,2 g/l) puis dans du milieu F12 (Boehringer). Après avoir ôté délicatement les méninges et isolé le cortex, celui-ci est dissocié mécaniquement à l'aide de ciseaux dans un milieu F12. Parallèlement, des explants de moelle épinière ont été réalisés et mis en culture selon le même principe. Le volume est ensuite ajusté à 30 ml de milieu F12 supplémenté avec 7,5% de sérum de veau fétal et 7,5% de sérum de cheval. Après 10 minutes de décantation, le surnageant est centrifugé 5 minutes à 4000 tr/min. Le culot obtenu est suspendu dans 10 ml de milieu complet. Les cellules sont étalées à une densité de $10^6$ cellules par boîte stérile de 35 mm de diamètre (Falcon) et maintenues à 37°C sous 7,5% de $CO_2$ et à 95% d'humidité. Alternativement, des lames avec alvéoles de culture de type Labtek® peuvent être utilisées, notamment pour une étude immunohistochimique ultérieure, après fixation de la culture au temps voulu. Le milieu de culture est changé tous les trois jours. Habituellement après 3 à 7 jours, lorsqu'une bonne différenciation des neurones corticaux est obtenue avec une organisation harmonieuse du tapis sous-jacent de cellules gliales et leptoméningées (un peu de pie-mère reste associée à l'échantillon de tissu cérébral utilisé), les cultures sont utilisées pour les tests de cytotoxicité vis-à-vis des cellules du système nerveux central.

Les échantillons à tester sont chauffés 30 min à 56°C, afin d'inactiver les protéines du complément et un éventuel virus enveloppé, puis centrifugés 10 minutes à 1500 tr/min. Eventuellement, le surnageant récupéré est dialysé à 4°C dans 2 fois 20 volumes de tampon D-PBS une première fois pendant 2 h, et une deuxième fois durant la nuit. L'échantillon à tester est aliquoté et conservé à -20°C. Un aliquot est décongelé pour les tests de cytotoxicité, mélangé selon la dilution voulue au milieu de culture des cellules cibles et le tout est replacé dans l'incubateur. Ici, les cellules cibles sont constituées de l'ensemble des cellules présentes dans la culture de l'explant de tissu cérébral.

Un effet cytotoxique important a été observé dans ces conditions avec des surnageants dilués au 1/4, dans le milieu de culture des explants sus-décrits, et prélevés entre le troisième et le dixième jour de culture de monocytes sanguins de patients atteints de SEP en poussée aigüe. Cet effet était observable au microscope optique utilisé pour l'examen des cellules en culture, dès la sixième heure suivant l'introduction de l'échantillon dilué dans le milieu de culture. L'effet était tout d'abord notable au niveau des cellules gliales, identifiées par leur morphologie tout à fait typique lors d'une observation régulière de la culture. On a ainsi observé un gonflement des oligodendrocytes qui finissaient par s'arrondir et se détacher du support de culture, ainsi qu'une vacuolisation des astrocytes accompagnée d'une régression importante de leurs extensions cytoplasmiques. A ce stade les neurones présents dans la culture étaient relativement conservés et ne présentent pas d'altération notable. Par la suite, après 24 heures environ dans ces conditions, le tapis glial étant complètement détruit, les neurones finissaient par dégénérer et se détâchaient à leur tour de la culture. Après 48 heures dans ces conditions il ne restait quasiment plus de cellules viables dans les puits de culture mis en présence des surnageants allant de J3 à J12, alors que l'effet des surnageants des mêmes cultures de monocytes de SEP en poussée aigüe prélevés à J1, J18 ou J21 présentaient un effet moindre, voire inexistant. Les surnageants des cultures de monocytes/macrophages sanguins de quelques témoins non-atteints de SEP n'ont, parallèlement, induit aucun effet cytotoxique dans les mêmes conditions.

**EXEMPLE 3: Test de cytotoxicité sur cultures d'explants de cerveau embryonnaire de rat.**

Des surnageants de cultures de monocytes/macrophages de SEP en poussée aigüe ou de témoin sain, ont été dilués au 1/4 dans le milieu de culture des explants de cerveau d'embryons de rat parvenus à un stade de différenciation avancée des neurones, puis incubés à 37°C comme décrit précédemment. Un exemple de l'effet cytotoxique sus-décrit observé au microscope optique dans les cultures d'explants embryonnaires est montré à la figure 1. La photographie A représente une culture de cellules de cerveau incubée 48 h avec un échantillon prélevé à J6 provenant d'un témoin sain. Les photographies B et C représentent deux cultures de cellules de cerveau incubées 24 h avec deux échantillons prélevés respectivement à J18 et à J6, provenant de la même culture de monocytes/macrophages de SEP en poussée aigüe. La photographie D représente une culture de cellules de cerveau incubée 48 h avec un échantillon prélevé à J6, provenant de la même culture de monocytes/macrophages de SEP en poussée aigüe que pour la photographie C.

Par ailleurs, un exemple de l'effet observé sur les différents types de cellules présents dans les cultures d'explants embryonnaires, par analyse immunocytologique au microscope à fluorescence, est montré aux figures annexées 2 et 3. Dans cet exemple, les surnageants de cultures de monocytes/macrophages de SEP en poussée aigüe ou de témoin sain, ont été dilués au 1/4 dans le milieu de culture d'explants de cerveau ou de moelle épinière d'embryons de rat cultivés sur lames Labtek® comme décrit précédemment. Les lames ont été fixées en temps voulu, après deux lavages en PBS, par incubation de 10 minutes dans un mélange volume à volume d'acétone et de méthanol à -20°C, puis incubées une nuit à +4°C avec la dilution adéquate d'un premier anticorps spécifique du type cellulaire à marquer, soit, un anticorps anti-neurofilaments (anticorps anti-NF Boehringer) pour les neurones, un anticorps anti-protéine gliale fibrillaire acide (anticorps anti-GFAP Boehringer) pour les astrocytes, et un anticorps anti-protéine basique de la myéline (anticorps anti-MBP Boehringer) pour les oligodendrocytes. Après deux lavages de 10 minutes en PBS suivis d'un lavage de 5 minutes en eau distillée, les lames ont été incubées une heure à température ambiante dans une dilution adéquate d'un second anticorps, spécifique des immunoglobulines de l'espèce ayant servi à produire le premier anticorps, et couplé à un fluorochrome. Après lavage des lames comme précédemment, celles-ci ont été montées pour être examinées au microscope à fluorescence avec la longueur d'onde adéquate. Dans la figure 2, les photographies A et B représentent, grossi 40 fois, le marquage par un anticorps anti-neurofilaments d'un explant de moelle épinière incubé avec un surnageant de culture de monocyte/macrophage de SEP en poussée aigüe prélevé à J9, pendant respectivement 12 et 48 heures. Dans la figure 2, les photographies C et D représentent, grossi 40 fois, un explant de moelle épinière incubé pendant 24 heures avec un surnageant de culture de monocyte/macrophage de SEP en poussée aigüe prélevé à J9, et marqué respectivement, avec un anticorps anti-neurofilaments et un anticorps anti-GFAP. Dans la figure 3, les photographies A et B représentent, grossi 40 fois, un explant de moelle épinière incubé pendant 24 heures avec un surnageant de culture de monocyte/macrophage de SEP en poussée aigüe prélevé à J6, et marqué respectivement, avec un anticorps anti-MBP et un anticorps anti-GFAP.

L'effet cytotoxique le plus précoce et le plus important dans ces cultures primaires de cellules du système nerveux central concerne donc à l'évidence les cellules macrogliales, à savoir les astrocytes et les oligodendrocytes.

## EXEMPLE 4: Activités cytotoxique et transcriptase inverse.

Le protocole de culture de monocytes/macrophages ayant d'abord été mis au point pour l'étude d'une expression rétrovirale dans la SEP **(45)**, on a testé dans quelques surnageants, l'activité transcriptase inverse selon les conditions auparavant déterminées pour l'étude ayant fait l'objet des travaux de Perron H. **(44)**, parallèlement à leur activité cytotoxique sur les cellules de cerveau cultivées en explants, comme décrit précédemment. Dans quelques surnageants de cultures de monocytes/macrophages de SEP prélevés entre J1 et J22, on observe une coïncidence relative entre le maximum d'activité gliotoxique retrouvée dans les surnageants de culture et le pic d'activité transcriptase inverse. Cependant, comme ni le chauffage pendant 30 minutes à 56°C, ni la congélation-décongélation à deux reprises, ni l'élimination du culot sédimenté par ultracentrifugation à 100 000 g pendant 2 heures, n'ont altéré l'effet cytotoxique des surnageants analysés, un effet direct dû à l'infection des cellules explantées par un rétrovirus présent dans les surnageants de cultures de monocytes/macrophages de SEP est invraisemblable.

La relative coincidence, dans quelques cultures de monocytes/macrophages de SEP, entre les activités transcriptase inverse et cytotoxique est montrée à la figure 4. Des surnageants de cultures de monocytes/macrophages de SEP, ont été dilués au 1/4 dans le milieu de culture d'explants de cerveau et incubés 24 heures selon le protocole sus-décrit avant estimation de l'effet cytotoxique par quantification microscopique de la déplétion cellulaire. Ces résultats montrent une relative coincidence entre l'activité transcriptase inverse et l'activité cytotoxique observée.

## EXEMPLE 5: Tests de cytotoxicité/gliotoxicité

Etant donnés les résultats sus-décrits, on a recherché un système standardisé d'étude et de quantification de l'effet cytotoxique observé. Après plusieurs évaluations, on a trouvé que les cultures continues d'astrocytes immortalisés **(50)** constituent un matériel adéquat pour la détection et quantification, sur des cellules gliales pures et homogènes, d'une activité cytotoxique telle que décrite précédemment.

Ainsi, la lignée continue d'astrocytes est maintenue dans un milieu DMEM-F12 (1/1), supplémenté avec 10% de SVF (sérum de veau foetal), dans un incubateur à 7,5% de $CO_2$, à 37°C et à 95% d'humidité. Les cellules sont mises en culture dans des plaques ou flacons de culture préalablement recouvertes de poly L-lysine à 5 µg/ml dans le PBS. La densité de passage est, en général, de $2x10^3$ cellules/$cm^2$. Dans ces conditions, les cellules sont cultivées deux jours, ou jusqu'à obtention d'un tapis cellulaire monocouche homogène, avant d'être utilisées pour les tests de cytotoxicité. Elles peuvent avantageusement être cultivées sur des plaques de cultures à 24 puits, permettant ainsi un travail en série sur de nombreux tests à effectuer. Pour les études ultérieures sur lame, elles peuvent être cultivées sur des lames de culture alvéolées de type Labtek® . De manière générale, les échantillons provenant de fluides biologiques à tester sont chauffés 30 min à 56°C, afin d'inactiver les protéines du complément et un éventuel virus enveloppé, puis centrifugés 10 minutes à 1500 tr/min, et le surnageant récupéré.

Chaque échantillon est ensuite dilué en proportion voulue dans le milieu de culture DMEM-F12 (1/1) à 10% de SVF des astrocytes sus-mentionnés, le milieu homogénéisé par pipettage ou agitation douce est déposé dans le flacon ou le puits de culture en remplacement du milieu de maintien. Les cellules sont ensuite replacées dans l'incubateur dans les conditions sus-décrites. L'effet cytotoxique vis-à-vis des cellules gliales que sont les astrocytes est ainsi apprécié en terme d'activité gliotoxique.

L'effet gliotoxique des dilutions des échantillons testés a été mesuré par trois techniques différentes qui se sont avèrées concordantes.

La première technique, appelée "L/D test" (test cellules vivantes / cellules mortes), est un dosage colorimétrique rapide, semi-quantitatif, permettant la détermination simultanée de cellules vivantes et mortes. Les cellules vivantes se distinguent par la présence d'une activité estérase intracellulaire. L'activité estérase est détectée par une fluorescence verte générée par l'hydrolyse enzymatique d'un substrat, la calcéine-AM. Les cellules mortes se distinguent par un marquage au niveau des acides nucléiques, par l'éthidium homodimère et seuls les noyaux des cellules dont les membranes nucléaires sont endommagées fluorescent en rouge. Après 72 h d'incubation à 37°C, les cellules sont rincées dans un tampon D-PBS et incubées 15 minutes à température ambiante et à l'abri de la lumière, en présence de 200 µl d'une solution de PBS contenant 2 µM de calcéine-AM et 4 µM d'éthidium homodimère. Une observation des cultures au microscope à fluorescence est ensuite effectuée dans un court délai. Les cellules vertes (vivantes) et rouges (mortes) sont comptées simultanément sur le même champ d'observation. Plusieurs champs sont observés (au moins trois) et la moyenne des comptages pour les cellules mortes et pour les cellules vivantes est prise comme résultat. L'activité gliotoxique (ou, plus généralement, cytotoxique) est exprimée selon la formule : % de cytotoxicité = (nombre moyen de cellules mortes / nombre moyen de cellules vivantes) x100.

La seconde technique consiste en un dosage colorimétrique au méthyltétrazolium des cellules vivantes. Le bromure de méthyltétrazolium (MTT) est un sel utilisé pour un dosage colorimétrique quantitatif **(51)**. Le MTT est un substrat des déshydrogénases mitochondriales qui, après réduction au niveau des cellules métaboliquement actives, donne un produit coloré, le formazan (violet). Il permet ainsi un marquage des cellules vivantes. Les cellules ($2x10^3$ cellules/cm$^2$) sont exposées 72 heures, à différentes dilutions de fractions purifiées (3 puits ou boîtes pour une dilution à tester). Elles sont ensuite rincées dans un tampon D-PBS puis incubées 3 h à 37°C dans 3 ml d'une solution MTT 0,5 mg/ml dans du DMEM-F12 (1/1). Le surnageant est éliminé et de l'isopropanol acide ($4x10^{-2}$ M HCl) est déposé sur les cellules afin de solubiliser les cristaux de formazan. Le lysat résultant est centrifugé 2 min / 6500 tr/min afin d'éliminer les débris cellulaires. Une lecture de densité optique est ensuite effectuée à 570-630 nm.

La troisième technique utilise un dosage radioactif au $^{51}$Cr qui permet la quantification des cellules mortes. Le $^{51}$Cr est un élément radioactif capable de pénétrer dans les cellules vivantes et qui n'est relargué dans le milieu extracellulaire que lors de la mort des cellules. La mesure de la radioactivité incorporée, après lavage des cellules mises en présence d'un milieu contenant du $^{51}$Cr, ainsi que de celle qui est relarguée par les cellules dans le milieu va permettre une quantification des cellules vivantes et mortes. Les astrocytes ($2x10^3$ cellules/cm$^2$) sont incubés 2 h à 37°C dans un milieu DMEM-F12 (1/1) contenant 20 µCi de $^{51}$Cr. Les cellules sont ensuite rincées 3 fois dans un milieu DMEM-F12, exposées à différentes dilutions de fractions purifiées (3 puits ou boîtes pour une dilution à tester) et incubées 72 h à 37°C. Le surnageant est récupéré et les cellules lysées avec une solution de NaOH 1M. On effectue ensuite un comptage en coups par minute (cpm) de la radioactivité (Compteur Gamma) présente dans le surnageant et dans le lysat, et on détermine un pourcentage de cytotoxicité calculé de la manière suivante : % cytotoxicité = [(cpm surnageant - cpm spontanée) / (cpm total- cpm spontanée)] x100. La radioactivité spontanée en cpm correspond au bruit de fond mesuré par le compteur sur un échantillon provenant d'une culture incubée avec un même fluide (LCR, sérum ou surnageant de culture de monocytes / macrophages) non gliotoxique et avec du $^{51}$Cr, dans les mêmes conditions que l'échantillon à tester.

Pour les études de reproductibilité et de fiabilité, plusieurs puits ont été incubés avec la même dilution du même échantillon gliotoxique et l'écart type obtenu a été pris en compte pour le caractère significatif des résultats des séries d'analyses ultérieures. Ce type de vérification peut être effectué à chaque changement de lot de cellules astrocytaires (décongélation d'une nouvelle ampoule) ou à chaque changement de lot de milieu de culture (lot de SVF, surtout). Dans les expériences mentionnées ici, un pourcentage de cellules mortes supérieur à 5% s'avère significatif d'un effet cytotoxique absent dans les conditions normales de culture.

**EXEMPLE 6: Cinétique de l'activité cytotoxique**

Des surnageants de cultures de monocytes / macrophages de SEP cytotoxiques sur des cultures primaires d'explants de cerveau embryonnaire, ainsi que des surnageants contrôles non-cytotoxiques, ont été testés sur des cultures de la lignée astrocytaire susmentionnée. La présence d'activité gliotoxique détectée par les astrocytes et visualisée par le "L/D test" a été retrouvée dans les surnageants préalablement cytotoxiques pour les cultures primaires et non dans ceux qui n'avaient pas eu cette propriété. On a ainsi pu étudier la cinétique, au cours du maintien en culture des mononocytes/macrophages, de l'expression de l'activité gliotoxique dans les surnageants de cultures provenant de patients atteints de SEP et de témoins. On a ainsi pu vérifier que cette activité n'était pas détectée tout au long des cultures provenant de témoins sains et, aussi, que son intensité fluctuait au cours des cultures "positives" provenant de patients atteints de SEP. En effet, plusieurs pics d'activité on pu être observés dans les quelques cas étudiés. Ces pics se situaient en général, entre le 6ème et le 9ème, puis entre le 15ème et le 18ème jour de culture (J6 à J9 et J15 à J18), indiquant ainsi une synthèse active dans la culture, puisque le surnageant y est entièrement prélevé deux fois par semaine à chaque changement du milieu de culture.

Un exemple d'une telle observation réalisée grâce à l'utilisation du test biologique mis au point est montré dans la figure 5. Dans cet exemple, les surnageants de cultures de monocytes / macrophages de SEP, ont été traités et utilisés comme décrit précédemment pour les tests de cytotoxicité / gliotoxicité sur la lignée d'astrocytes sus-citée avec une dilution au 1/10ème dans le milieu de culture et incubés 72 heures selon le protocole sus-décrit avant mesure de l'effet gliotoxique par la méthode du "L/D test".

**EXEMPLE 7: Mise en évidence d'une activité gliotoxique dans des fluides biologiques de patients atteints de SEP.**

Par la suite, ce test biologique a été utilisé pour rechercher une éventuelle activité gliotoxique dans le liquide céphalorachidien (LCR) et le sérum. Ainsi, les inventeurs ont pu mettre en évidence une activité gliotoxique significative dans les LCR de patients atteints de SEP et non dans des LCR de témoins atteints, par exemple, d'hydrocéphalie à pression normale (HPN). La même constatation a été effectuée avec les sérums correspondants, cependant avec un activité gliotoxique relative plus faible que dans les LCR, ce qui est en faveur d'une production intrathécale du facteur portant cette activité.

Un exemple de l'utilisation du "L/D test", pour visualiser la détection par les cibles astrocytaires d'un facteur gliotoxique dans les échantillons mélangés au milieu de culture, est montré dans la figure 6 annexée. Dans cet exemple, un LCR de SEP rémittante en poussée a été dilué au 1/10 dans le milieu de culture, incubé 72 heures et examiné au microscope à fluorescence, le tout selon le protocole sus-décrit pour le "L/D test". La photographie A, représente une visualisation des cellules vivantes avec un système optique adéquat pour les émissions lumineuses de la fluorescéine (la barre représente 25 micronmètres). La photographie B, représente une visualisation des cellules mortes avec un système optique adéquat; la barre représente 25 micronmètres. La photographie C, représente une visualisation simultanée des cellules vivantes et mortes avec un système optique adéquat pour les émissions lumineuses des deux fluorophores (485-500 nm); la barre représente 25 micronmètres.

Une fois ce test de quantification mis au point, les inventeurs ont analysé des filtres préparés par la firme Pall et utilisés dans des essais thérapeutiques sur des patients atteints de SEP **(52)**. Ces essais consistent à filtrer le liquide céphalorachidien au travers de filtres ayant notamment une affinité pour les glycoprotéines et sélectionnés par la Société PALL. On a obtenu des filtres après filtration du LCR de quelques cas de SEP et remis en solution les protéines adsorbées sur les filtres à l'aide d'une solution à 1% de SDS. Après mise en conditions physiologiques des échantillons, il a pu être montré qu'une forte activité gliotoxique évaluée par le présent test biologique sur lignée astrocytaire, est associée aux protéines préalablement retenues sur ces filtres.

Un exemple de l'utilisation du test biologique pour détecter et quantifier une activité gliotoxique dans les fluides biologiques, notamment dans les LCR de patients atteints de SEP, est présenté dans le tableau 1 annexé, où sont montrés les effets de la filtration du LCR sur le filtre fourni par la Société PALL.

Dans ce tableau, les LCR ont été filtrés sur filtre PALL ayant le même type de support filtrant que ceux utilisés in vivo dans des essais thérapeutiques. L'activité gliotoxique de chaque filtrat mesurée selon le test biologique sur astrocytes et le résultat comparé à l'activité gliotoxique du LCR natif d'avant filtration, avec le caractère significatif statistique de la différence observée sur une série de 10 mesures, y sont présentés. Dans cet exemple, les LCR ont été dilués au 1/10 dans le milieu de culture et incubés 72 heures selon le protocole sus-décrit pour le "L/D test". La valeur moyenne du pourcentage de cellules mortes a été obtenue, pour chaque échantillon, sur 5 champs choisis au hasard sur deux puits incubés en parallèle avec le même échantillon. Une différence statistiquement significative existe entre les LCR de SEP et les filtrats obtenus après filtration. Les diagnostics de SEP sont, sauf précision contraire, des diagnostics de SEP certaines selon les critères de Poser **(49)**.

Cet exemple montre qu'il existe des moyens physicochimiques d'éliminer une partie au moins dudit facteur gliotoxique des fluides biologiques de patients atteints de SEP, ou d'autres maladies dans lesquelles ledit facteur gliotoxique ou encore ladite activité gliotoxique serait détectée in vivo. Ces résultats confirment aussi la réalité moléculaire de ladite activité gliotoxique, ainsi que l'intérêt des techniques de détection et de dosage qui sont des objets de la présente invention.

Un autre exemple de l'utilisation du test biologique mis au point par les inventeurs, pour détecter et quantifier une activité gliotoxique dans les fluides biologiques, notamment dans les LCR de patients atteints de SEP, est présenté dans le tableau 2 annexé. Dans cet exemple, les LCR ont été dilués au 1/20 dans le milieu de culture selon le protocole sus-décrit pour le test colorimétrique au MTT et incubés 96 heures. Chaque résultat représente la moyenne de deux expériences séparées et représentant chacune une série de 5 puits indépendants, soit, finalement, une moyenne de 10 valeurs par LCR testé.

Les échantillons de LCR provenant de patients atteints de forme rémittante de SEP, ont été prélevés au moment de poussées cliniques. La différence des moyennes de cytotoxicité dans les deux sous-populations de SEP, rémittantes versus chroniques progressives, est statistiquement significative, ce qui indique a priori que la quantification de l'activité gliotoxique du LCR permet de corréler l'activité clinique de la maladie. Les diagnostics de SEP, sont, sauf précision contraire, des diagnostics de SEP certaines selon les critères de Poser **(49).**

Les résultats présentés dans les tableaux 1 et 2 ainsi que d'autres similaires, ont ainsi permis aux inventeurs de mettre en évidence l'existence d'une activité gliotoxique très significative dans les LCR de patients atteints de SEP et une variation de son intensité en fonction du stade clinique et/ou de la forme évolutive. Ces résultats confirment l'intérêt du test biologique mis au point sur lignée astrocytaire, en combinaison avec une technique de quantification pouvant être par exemple, l'une des trois techniques sus-décrites ("L/D test", dosage colorimètrique au MTT et dosage

du $^{51}$Cr relargué) dans les études pronostiques et/ou les suivis thérapeutiques de pathologies dans lesquels une telle activité cytotoxique, et notamment, gliotoxique peut être détectée, voire dans le diagnostic d'une maladie telle que la SEP.

**EXEMPLE 8: Activité cytotoxique observée sur d'autres types cellulaires.**

Par ailleurs, la spécificité de l'activité cytotoxique observée a aussi été évaluée, et quantifiée par le "L/D test", sur des cultures de différents autres types cellulaires. Avec des échantillons s'étant préalablement révélés cytotoxiques vis-à-vis des cellules gliales, soit en culture d'explants de cerveau embryonnaire de rat, soit sur lignée astrocytaire, aucune cytotoxicité directe n'a été détecté sur fibroblastes, myoblastes, cellules musculaires de pattes de souris et sur cellules endothéliales. Une cytotoxicité nettement plus faible, d'environ 10% relativement aux astrocytes sus-cités, a été observée sur les cellules de Schwann de nerf sciatique. Il est intéressant de noter ici que les cellules de Schwann sont responsables de la myélinisation des nerfs périphériques, au même titre que les oligodendrocytes sont responsables de la myélinisation normale dans le système nerveux central. Dans les cultures d'explants de cerveau embryonnaire, les quelques cellules leptoméningées présentes ne semblent pas affectées directement, de même que les neurones qui ne semblent affectés qu'après modification importante du tapis nourricier de cellules gliales. Les monocytes sanguins mis en culture et différenciés en macrophages après adhésion au support de culture, ne semblent pas affectés non plus puisqu'ils persistent en culture et cela, même en présence de plusieurs pics successifs d'activité gliotoxique relarguée dans le surnageant au cours des cultures macrophagiques (voir figure 5). Quant aux lymphocytes, des résultats contradictoires ont été obtenus avec des cultures de cellules exprimant l'antigène CD4, à savoir qu'avec le même échantillon gliotoxique comparé au même échantillon témoin non gliotoxique, une mort cellulaire significativement accrue a été observée dans certaines cultures alors qu'une prolifération cellulaire était observée dans des cultures provenant d'autres individus donneurs de lymphocytes.

Ces observations sur les lymphocytes s'apparentent aux effets décrits pour des molécules aux propriétés superantigèniques **(26, 27).**

**EXEMPLE 9: Caractérisation cytologique des effets cytotoxiques.**

Ces données obtenues avec les lymphocytes ont conduit les inventeurs à préciser au préalable les effets cytologiques liés à la cytotoxicité, sur les astrocytes utilisés dans les tests biologiques de gliotoxicité. Une étude des filaments intermédiaires des astrocytes susmentionnés a révélé un effet important des fluides gliotoxiques sur l'organisation du cytosquelette astrocytaire, après incubation des cellules selon le protocole décrit précédemment pour les tests de gliotoxicité. En effet, alors qu'aucune modification n'est observée avec les fluides témoins non-gliotoxiques testés en parallèle, une désorganisation drastique des filaments de vimentine et de GFAP est observée dans les cultures d'astrocytes mises en présence d'une activité gliotoxique significative provenant de fluides biologiques de patients atteints de SEP (culture de monocytes / macrophages ou LCR). Ce phénomène avait aussi été observé sur les cultures primaires d'explants de système nerveux embryonnaire, dans lesquels les astrocytes étaient spécifiquement marqués par un anticorps anti-GFAP.

Un exemple de cette désorganisation des filaments intermédiaires de vimentine et de GFAP sur les astrocytes en cultures mis en présence de fluides gliotoxiques issus de patients atteints de SEP, est présenté dans la figure 7. Dans cet exemple, la détection de la GFAP a été effectuée sur culture primaire de cellules cultivées sur lames de type Labtek® . Après incubation d'une heure et de 24 heures avec le mileu de culture contenant une dilution au 1/20 d'un surnageant gliotoxique de culture de monocytes / macrophages de SEP, les cellules adhérentes sur la lame sont rincées 2 fois dans PBS puis fixées dans du paraformaldéhyde 4%, 20 min à 37°C. Après 2 rinçages dans du PBS, elles sont incubées 2 fois 5 minutes dans une solution de blocage PBS / 1% lait. Le premier anticorps est polyclonal, développé chez le lapin. L'anticorps est dilué au 1/50[e] dans la solution de blocage. L'incubation dure 1 h à 37°C. Après la première incubation , on effectue une série de rinçages de 5 minutes dans du PBS puis dans la solution de blocage, afin d'éliminer les anticorps non fixés. Le système révélateur utilise la fluorescéine couplée à une immunoglobuline anti-lapin diluée au 1/100[e] dans la solution de blocage. L'incubation dure 1 h à 37°C. Le montage des préparations est fait avec du moviol pour éviter la décroissance de la fluorescence au moment de l'observation. Les lames sont conservées à l'obscurité à 4°C. Les observations sont faites au microscope à fluorescence. La détection de la vimentine a été effectuée sur lignée astrocytaire cultivée sur lames de type Labtek® , après incubation de 24 heures avec, soit une dilution au 1/50 d'un surnageant gliotoxique de culture de monocytes/macrophages de SEP, soit un milieu de culture normal. Après 24 heures d'incubation, les cellules ($1 \times 10^3$ cellules/cm$^2$) sont rincées 2 fois dans du PBS, puis fixées 10 min à -20 °C avec de l'acétone. Après 3 rinçages dans du PBS, elles sont incubées 3 fois 5 minutes dans une solution de blocage PBS / 5% SVF à température ambiante. Le premier anticorps est polyclonal, développé chez la chèvre. L'anticorps est dilué au 1/50[e] dans la solution de blocage. L'incubation est de 2 h à 37°C. Après 3 rinçages de 5 minutes dans PBS puis dans la solution de blocage, les cellules sont incubées 1 h à 37°C avec un deuxième anticorps dirigé contre les IgGs de chèvre, couplé à la fluorescéine et dilué au 1/200ᵉ dans la solution de blocage. Les photographie A et B représentent un marquage de la GFAP après, respectivement, 1 et 24 heures d'incubation en présence de l'échantillon gliotoxique dilué. Les photographies C et D représentent un marquage de la vimentine des astrocytes en lignée incubés 24 heures avec, respectivement, un milieu normal et un milieu contenant l'échantillon gliotoxique dilué.

De plus, les inventeurs ont étudié l'aspect de l'ADN cellulaire extrait de cultures d'astrocytes cultivés en milieu normal et cultivés pendant des temps croissants en présence d'échantillons gliotoxiques issus de SEP. Il a alors été constaté que l'ADN issu de cultures soumises à une activité gliotoxique présentaient une fragmentation de l'ADN cellulaire dont l'intensité augmentait en fonction de la durée d'incubation des cellules, alors que l'ADN des cellules incubées en milieu sans activité gliotoxique restait homogène à un très haut poids moléculaire.

Ces observations sont compatibles avec un processus d'apoptose tel que peuvent en induire des superantigènes, sur des cellules lymphoïdes notamment.

**EXEMPLE 10: Effet dose-réponse**

Par la suite, afin d'évaluer la possibilité que cette activité gliotoxique détectée soit portée par une molécule, ou par un facteur dont la représentation moléculaire pourrait s'avérer plus complexe, présent dans les fluides testés, les inventeurs ont d'abord évalué, à l'aide des tests biologiques mis au point et décrits précédemment, la réalité d'un effet dose-réponse sur la cytotoxicité induite par des échantillons de référence. Un effet dose-réponse compatible avec une proportionalité directe de la mort cellulaire par rapport à la dilution, et avec un effet de saturation du système de détection astrocytaire aux fortes concentrations, a été observé après visualisation de l'effet par le "L/D test".

Un exemple de cet effet dose réponse observé avec un fluide biologique gliotoxique est présenté dans la figure 8. Dans cet exemple, un surnageant de culture de monocyte/macrophage de SEP a été mélangé, en dilutions successives, au milieu de culture des astrocytes selon le protocole sus-décrit pour le "L/D test".

Les inventeurs ont ainsi caractérisé une activité cytotoxique et plus particulièrement gliotoxique dans les cultures de monocytes / macrophages sanguins, le LCR et le sérum de patients atteints de SEP, parallèlement à la mise au point d'un procédé de détection de ladite activité cytotoxique sur cultures primaires d'explants de cerveau ou de moelle épinière d'embryons de rat, et de ladite activité gliotoxique sur lignées astrocytaires en culture, ainsi encore que la mise au point d'un procédé de quantification de ladite activité gliotoxique couplé au procédé de détection sur astrocytes en culture.

S'étant d'abord attaché à la mise en évidence de cette activité gliotoxique in vivo chez les patients atteints de SEP et aux moyens de son dosage systématique et standardisé, les inventeurs se sont attachés ensuite à tenter de relier cette activité avec une molécule particulière, ou un facteur représenté par un ensemble de molécules définies, dans les fluides testés.

**EXEMPLE 11: Caractérisation du facteur cytotoxique/gliotoxique**

Les inventeurs ont d'abord observé que l'activité biologique définie en tant qu'activité gliotoxique par les procédés décrits plus haut, persistait dans les échantillons gliotoxiques placés dans un bain-marie à +56°C pendant une demi-heure mais était abolie lorsque la température était de +100°C pendant 15 minutes, persistait après la congélation jusqu'à -80°C de l'échantillon suivie de sa décongélation jusqu'à +37°C. Après centrifugation à 100 000 g pendant 2 heures et élimination du culot de matériel sédimenté, l'activité d'échantillons préalablement reconnus gliotoxiques selon les procédés décrits dans la présente invention, était toujours présente dans le surnageant et s'apparente donc à une facteur soluble non particulaire. De même, l'incubation d'échantillons gliotoxiques dont la quantité totale de protéine a été préalablement déterminée par la technique de Bradford **(53)** en présence de trypsine, de pronase, de protéinase K, ou d'un mélange de N-glycosidase F et de neuraminidase, dans des conditions suffisantes pour une hydrolyse enzymatique complète des liaisons peptidiques ou des N-glycosylations présentes dans l'échantillon, n'abolit pas leur activité gliotoxique telle que décrite précédemment.

Par la suite, les inventeurs ont réalisé la séparation et le fractionnement des différents composés d'échantillons préalablement avérés gliotoxiques par les procédés décrits dans la présente invention. Pour l'ensemble de ces travaux, les échantillons sont préalablement chauffés 30 min à 56°C et centrifugés 10 min à 1500 tr/min, puis le surnageant est récupéré et, éventuellement, dialysé à 4°C dans 2 fois 20 volumes de tampon D-PBS, une première fois pendant 2 h, et une deuxième durant la nuit. Le surnageant ainsi recueilli constitue l'échantillon sur lequel les différentes opérations sont effectuées. De plus, les fractions issues des échantillons dont les composantes moléculaires ont été séparées par différents procédés sont traitées de manière à éliminer les éventuelles molécules toxiques introduites dans les milieux recueillis par les procédés utilisés, et de manière à replacer les molécules organiques en solution dans des conditions physiologiques compatibles avec les procédés de détection et de quantification de l'activité biologique gliotoxique tels que décrits dans la présente invention. Pour ce, les échantillons sont lyophilisés, resuspendus dans 2,5 ml d'eau distillée stérile, déposés sur une colonne de chromatographie de type NAP-25 (Pharmacia) préalablement lavée dans 10 volumes de tampon D-PBS, puis élués avec 3,5 ml de tampon D-PBS et utilisés comme tels pour les test d'activité gliotoxique.

Ainsi, pour étudier la charge ionique du facteur gliotoxique tel que précédemment défini, des échantillons gliotoxiques selon les critères sus-définis et issus de surnageants de culture de monocytes / macrophages et de LCR de SEP, ont été passés avec un débit de 60 ml/heure sur colonne de chromatographie FPLC de type DEAE-Sépharose CL-6B (Pharmacia) équilibrée dans un tampon A (50 mM Tris-HCl à pH 8,8.) Dans ces conditions la fraction portant l'activité gliotoxique présente dans l'échantillon d'origine est éluée avec une force ionique entre 0,12 M et 0,2 M de NaCl de tampon A.

Pour étudier une éventuelle analogie physicochimique avec certaines sérine-protéases qui ont une forte affinité pour des supports de chromatographie liquide de type Blue-Sépharose (Pharmacia), des échantillons gliotoxiques selon les critères sus-définis, issus de surnageants de culture de monocytes / macrophages et de LCR de SEP et préalablement dialysés à 4°C dans 2 fois 20 volumes de tampon B (50 mm Tris, pH 7.2) et contenant 5 mg de protéines par ml, ont été déposés sur colonne FPLC de type Blue-Sépharose CL-6B (Pharmacia). Après élution avec 0,1 M de KCl dans le tampon B, aucune activité gliotoxique n'a été retrouvée dans l'éluat, alors que la fraction portant l'activité gliotoxique a été recupérée avec un rendement d'activité proche de 70%, par élution avec 1,5 M de KCl dans le tampon B. L'analyse a aussi révélé que la sérumalbumine était aussi éluée dans cette même fraction. Cependant, l'activité protéasique de l'éluat a été testée par incubation de celui-ci avec de l'"azocaséin" (Sigma) ou de l'"azocoll" (Sigma), dans un tampon D-PBS pH 7,5 à 37°C pendant 2 heures, sans qu'aucune activité protéolytique de l'échantillon ainsi préparé et élué ne puisse être mise en évidence.

Pour étudier l'association éventuelle d'un tel facteur gliotoxique avec les immunoglobulines de type IgG, des échantillons gliotoxiques selon les critères sus-définis et issus de surnageants de culture de monocytes / macrophages et de LCR de SEP, ont été déposés sur colonne FPLC de type protéine A-Sépharose CL-4B (Pharmacia) préalablement lavée avec 5 volumes de D-PBS. La teneur en protéine A du gel gonflé était égale à 2 mg/ml et la capacité de fixation d'IgG humaines était de l'ordre de 20 mg d'IgG / ml de gel (volume du gel 1,5 ml). La fraction dépourvue d'IgG a été récupérée par élution avec du D-PBS et contenait l'activité gliotoxique, alors que la fraction enrichie en IgG éluée avec un tampon glycine-HCl 50 mm à pH 3,0 ne contenait pas d'activité gliotoxique telle que définie précédemment quand les échantillons provenaient de LCR ou de surnageants de cultures de monocytes-macrophages effectuées dans les conditions décrites précédemment. Dans le cas d'échantillons provenant de sérums humains, une faible activité gliotoxique a été retrouvée dans la fraction enrichie en IgG, et ce, aussi bien pour des sérums de SEP en poussée prélevés en même temps qu'un LCR s'avèrant gliotoxique, que pour des sérums de témoins atteints par exemple, d'hydrocéphalie à pression normale (HPN), dont le LCR prélevé en même temps ne présentait aucune activité gliotoxique. Cependant, l'étude des sérums d'HPN ou de témoins sains n'a révélé aucune activité gliotoxique dans la fraction dépourvue d'IgG éluée par le D-PBS alors que la quasi-totalité de l'activité gliotoxique importante des sérums de patient atteints de SEP en poussée a été retrouvée dans cette fraction dépourvue d'IgG éluée par le D-PBS. Ceci, suggère qu'il existe un ou des composants gliotoxiques dans le sérum de personnes saines qui sont différents du facteur gliotoxique qui a été mis en évidence par les inventeurs de la présente invention, d'activité nettement plus faible et que l'on retrouve élués dans la fraction enrichie en IgG après passage sur colonne de protéine A-Sépharose dans les conditions sus-décrites. Ceci s'apparente vraisemblablement à une gliotoxicité non spécifique liée à des protéines actives du sérum tel que cela a déjà été rapporté **(54)**. Pourtant, la faible quantité de sérum humain provenant de donneurs sains ayant un groupe sanguin AB positif, présent dans les cultures de monocytes / macrophages ne semble pas produire, dans ces conditions d'analyse, cette gliotoxicité additive ainsi recueillie avec l'élution des IgG. Ceci peut provenir de l'effet de dilution qui, du fait de la faible activité de cette composante, la rend indétectable par nos procédés, ou bien, d'un effet d'inactivation de cette composante au cours de la culture de monocytes / macrophages dans les conditions sus-décrites. Si l'on ajoute à ce fait, l'absence de gliotoxicité détectable dans ces conditions après une même analyse d'échantillons non-gliotoxiques provenant de LCR de témoins atteints d'HPN ou provenant de surnageants de cultures de monocytes / macrophages de personnes saines, ces résultats confirment l'originalité de l'activité gliotoxique et, par là du facteur gliotoxique associé, constituant des objets de la présente invention, au regard des activités gliotoxiques pouvant être retrouvées en l'absence de processus pathologique dans les fluides biologiques d'individus apparemment sains.

Pour étudier le poids moléculaire dudit facteur gliotoxique, les inventeurs ont parallèlement et successivement analysé des échantillons gliotoxiques selon les critères sus-définis et issus de surnageants de culture de monocytes / macrophages, de filtres PALL ayant servi à filtrer in vivo le LCR de patients SEP et de LCR de SEP, sur colonne FPLC de type Superose 12 (Pharmacia) et sur électrophorèse en gel de polyacrylamide en présence de SDS (SDS-PAGE). Après analyse sur colonne Superose 12, les fractions contenant l'activité gliotoxique ont été éluées après un volume d'élution correspondant à un poids moléculaire d'environ 17 KD, par correspondance avec la courbe de référence des volumes d'élution de protéines globulaires standard. Plus précisément, après recueil de fractions plus rapprochées dans cette zone d'élution, il a pu être observé deux pics d'activité gliotoxique dissociés, à des volumes d'élution correspondant à un poids moléculaire d'environ 21 KD et d'environ 16,8 KD. Une élution similaire d'un même échantillon dans un tampon auquel de l'urée 8 M a été ajoutée donne des résultats identiques indiquant qu'il n'y a pas d'associations multimériques des composants élués dans des conditions de tampons physiologiques.

Après analyse par SDS-PAGE en une dimension, les bandes protéiques contenant l'activité gliotoxique, après élution du gel et remise en conditions physiologiques, ont été retrouvées dans une portion de gel correspondant à un poids moléculaire d'environ 17 KD, et dans certaines conditions d'analyse et de richesse en matériel biologique analysé, dans une portion supplémentaire d'environ 21 KD. En parallèle, les autres bandes protéiques ainsi que différentes régions du gel sans protéines ont été testées sans qu'aucune activité gliotoxique significative ne puisse y être décelée. L'analyse en deux dimensions de la bande protéique gliotoxique extraite du gel vers 17 KD, a montré l'existence de deux tâches majeures à 17 KD présentant, entre environ un pH 6 et un pH 7, un point isoélectrique différent, et d'une tâche mineure à 18 KD ayant un point isoélectrique légèrement plus basique, supérieur à un pH d'environ 7.

Un exemple d'analyse par électrophorèse à une et deux dimensions sur gel, des composants protéiques d'une fraction gliotoxique obtenue après passage sur colonne DEAE est présenté dans les figures 9A et 9B. Dans la figure 9A, on peut voir la photographie d'un gel d'acrylamide à une dimension coloré au bleu de Coomassie provenant d'un mélange d'échantillons gliotoxiques élués à partir d'une dizaine de filtres PALL ayant servi à la filtration in vivo du LCR d'une série de patients atteints de SEP. Ces filtres sont ouverts mécaniquement et les filtres lavés en présence de SDS 1%, puis les éluats remis en tampon physiologique à l'aide, notamment, d'un passage sur colonne NAP-25 ainsi que décrit précédemment. Les filtrats ainsi traités sont testés pour leur activité gliotoxique sur un aliquot et passés en mélange sur colonne DEAE-Sépharose. La fraction contenant l'activité gliotoxique est dessalée sur colonne NAP-25 et concentrée par évaporation avant d'être déposée en deux parties, l'une sur un gel SDS-PAGE une dimension (figure 9A) et l'autre sur un gel SDS-PAGE à deux dimensions. Une photographie d'un tel gel à deux dimensions est présentée dans la figure annexée 9B. La bande visualisée avec un poids moléculaire apparent de 17 KD en une dimension s'avère être la seule bande protéique visualisée portant une activité gliotoxique sur le gel montré en 9A et l'on peut voir, sur le gel en deux dimensions (9B) que cette bande de 17 KD environ se sépare en trois taches de points isoélectriques différents, la troisième tache sur la droite ayant un poids moléculaire apparent légèrement supérieur aux deux taches de gauche.

Un exemple d'analyse avec une colonne FPLC superose 12 est présenté dans les figures 10A et 10B. Dans cet exemple, des surnageants de culture de monocytes / macrophages d'un patient atteint de SEP prélevés entre le 6ème et le 16ème jour de culture, représentant un volume de 20 ml et 140 mg de protéines, ont d'abord été passés avec un débit de 60 ml/heure sur colonne de chromatographie FPLC de type DEAE-Sépharose CL-6B (Pharmacia) équilibrée dans un tampon A (50 mM Tris-HCl à pH 8,8). Parallèlement, une colonne Superose 12 a été équilibrée avec un tampon C, Tris HCl 50 mM, pH 6,8, et calibrée en éluant un mélange de protéines globulaires de poids moléculaire connu dans ce même tampon C. La fraction portant théoriquement l'activité gliotoxique présente dans l'échantillon d'origine a été éluée de la colonne DEAE-Sépharose avec une force ionique entre 0,12 M et 0,2 M de NaCl de tampon A, dans un volume de 15 ml contenant 39 mg de protéines. Un tiers de cette fraction, contenant 13 mg de protéines, a été ensuite déposée sur la colonne superose 12 dans un tampon sans urée (Fig 10A) et un autre tiers sur une colonne identique, en tampon 8 M urée (Fig 10B). Pendant élution dans le tampon C, 40 fractions ont été recueillies et une mesure continue de l'absorption à 280 nm, pour doser les protéines dans l'éluat, a été enregistrée avec une forte sensiblité de détection (R = 1,2). L'activité gliotoxique des différentes fractions recueillies a été testée selon notre test biologique sur lignée astrocytaire et quantifiée, après 72 h d'incubation, par la technique du "L/D test". Pour ce faire, les fractions ont été lyophilisées, resuspendues dans 2,5 ml d'eau distillée stérile, déposées sur colonne de chromatographie de type NAP-25 (Pharmacia) préalablement lavée dans 10 volumes de tampon D-PBS, puis élués avec 3,5 ml de tampon D-PBS et utilisées comme tels pour les test d'activité gliotoxique. Dans les figures 10A et 10B, la courbe en trait noir continu représente la densité optique à 280 nm et la courbe en trait pointillé représente l'activité gliotoxique. Les correspondances entre le volume d'élution (Ve) et le poids moléculaire apparent, ont été calculées en fonction de la calibration préalablement réalisée avec des protéines de référence et est mentionnée au sommet des pics d'activité gliotoxique. Il est à noter qu'une activité gliotoxique faible est retrouvée dans une fraction correspondant à un poids moléculaire d'environ 110 KD. Cependant la similitude des profils d'élution en présence et absence d'urée 8 M est en faveur d'une composition monomérique du facteur élué au niveau de ces différents poids moléculaires. Ainsi donc dans cet exemple, l'activité gliotoxique spécifique aux facteurs de poids moléculaires apparents d'environ 21 KD et d'environ 17 KD peut être mise en évidence et individualisée.

Les résultats obtenus par chromatographie FPLC sur colonne Superose 12 et par analyse électrophorétique, montrent que le facteur gliotoxique est au moins constitué d'une protéine ou molécule associée de poids moléculaire de 17 KD et, d'une protéine ou molécule associée de poids moléculaire de 21 KD. Le fait que les poids moléculaires apparents où est retrouvée l'activité gliotoxique soient identiques dans les deux types de techniques (FPLC et électrophorèse) suggère qu'il s'agit de protéines globulaires. De plus une étude comparative de gels SDS-PAGE en présence ou en l'absence de β-mercaptoéthanol ayant par ailleurs donné des profils de migration identiques, il s'agit vraisemblablement d'homomères. Le fait que l'activité gliotoxique soit retrouvée, parmi toutes les fractions de chromatographie et toutes les portions de gel analysées, à deux poids moléculaires différents d'environ 17 KD et d'environ 21 KD, peut s'expliquer par deux molécules sans homologie significative, ou par l'existence de glycosylations ou de toutes modifications post-traductionnelles différentes sur un même support protéique, ou encore par l'existence d'un pro-peptide ou d'une partie peptidique quelconque qui, après clivage d'une protéine de 21 KD environ, génère une protéine d'environ 17 KD.

Aussi, pour étudier la glycosylation éventuelle dudit facteur gliotoxique, des échantillons gliotoxiques selon les critères sus-définis et issus de surnageants de culture de monocytes / macrophages, de sérums, de LCR de SEP, et de filtres ayant servi à filtrer in vivo le LCR de patients SEP, ont été analysés sur colonne FPLC de type Concanavaline A-Sépharose (Con A-Sépharose, Pharmacia). Pour cette étude, les échantillons biologiques gliotoxiques ont été préalablement passés sur colonne FPLC de type DEAE-Sépharose ou de type protéine A-Sépharose. Les fractions contenant l'activité gliotoxique soit, respectivement, une fraction éluée vers 0,2 M de NaCl et une fraction éluée avec du D-PBS, sont ensuite utilisées pour la chromatographie FPLC sur colonne de type Con A-Sépharose. Dans ces conditions, les fractions éluées de la colonne Con A-Sépharose, soit avec du D-PBS contenant jusqu'à 500 mM de NaCl, soit ensuite avec 200 mM de D-glucopyranoside qui présente une affinité compétitive pour la Con A, n'ont présenté aucune activité gliotoxique. L'activité gliotoxique présente dans l'échantillon d'origine a été retrouvée concentrée dans la fraction éluée, après les deux élutions précités, avec un tampon glycine-HCl 50 mM pH 3,0 avec du $Ca^{++}$ 1 mM et du $Mn^{++}$ 1 mM. Cette fraction correspond à des molécules ayant une haute affinité pour la Con A, ce qui correspond en général à des molécules fortement glycosylées. L'analyse de cette fraction en SDS-PAGE une dimension révèle que l'activité gliotoxique est retrouvée associée à deux bandes protéiques de 17 KD et de 21 KD, respectivement.

Un exemple d'analyse en gel SDS-PAGE unidimensionnel d'une purification utilisant une colonne FPLC de type DEAE-Sépharose Con A-Sépharose est présenté dans la figure 11. Dans cet exemple, 27 ml de surnageant, contenant 100 mg de protéines, de culture de monocyte / macrophage d'un patient atteint de SEP ont été passés sur colonne DEAE-Sépharose. et l'éluat obtenu avec une force ionique entre 0,12 et 0,20 M de NaCl a été recueilli dans un volume de 10 ml contenant 32 mg de protéines. Cette fraction a été passée ensuite sur colonne de type Con A-Sépharose. Une première élution a été réalisée avec du D-PBS 500 mM, une deuxième élution a été réalisée avec un tampon D-PBS contenant 200 mM de D-glucopyranoside, une troisième élution a été réalisée avec un tampon glycine HCl 50 mM pH 3,0 avec du Ca++ 1 mM et du Mn++ 1 mM et un éluat de 6,4 ml contenant 0,17 mg de protéines a été recueilli.

Après cette série de purifications où la gliotoxicité de tous les échantillons intermédiaires a été dosée, 79% de la gliotoxicité du surnageant d'origine a été retrouvée dans la fraction éluée en tampon glycine à pH 3 de la colonne Con A-Sépharose, avec un rendement de purification protéique de 465 fois.

Sur un gel SDS à 10% d'acrylamide, des échantillons de chaque étape ont été déposés successivement dans des puits parallèles, correspondant à une quantité protéique de 2,2 mg pour la troisième fraction éluée sur Con A-Sépharose. Il est à noter que des rendements encore meilleurs ont pu être obtenus pour les mêmes étapes avec du LCR de patients atteints de SEP.

De plus, à partir de la troisième fraction éluée sur Con A-Sépharose, une électrophorèse préparative SDS-PAGE a été effectuée en parallèle, différentes bandes ont été découpées dans le gel non coloré à la hauteur de toutes les bandes protéiques colorées au bleu de Coomassie dans un puits de référence, ainsi que dans quelques régions sans protéine. Pour analyser la gliotoxicité de chaque bande ainsi découpée, les morceaux de gel ont été écrasés et homogénéisés dans du D-PBS à 0,2% de SDS, incubés à 37°C pendant 30 minutes puis centrifugés à 100 000 g pendant 6 minutes. L'opération est répétée deux fois et les surnageants des deux centrifugations sont mélangés et passés sur colonne DEAE-Sépharose. L'éluat obtenu par élution avec un tampon D-PBS 200 mM NaCl est recueilli et utilisé pour les tests de gliotoxicité. Dans ces conditions, le SDS est retenu sur la colonne et l'éluat est physiologiquement compatible avec les cultures cellulaires. Dans ces extraits de gel d'électrophorèse, une activité gliotoxique significative n'a été retrouvée que dans les bandes découpées dans des zones de poids moléculaire d'environ 17 KD et 21 KD. Afin de vérifier le profil protéique de ces deux extraits, ils ont été déposés sur le gel analytique en parallèle avec les fractions de chromatographie précédentes. Sur la figure 11 présentant les résultats de la révélation des protéines migrées après électrophorèse, en partant de la gauche, la première colonne représente une série de protéines standard de poids moléculaires connus et indiqués sur la gauche, la deuxième colonne surmontée d'un "S" représente le surnageant de départ, la troisième colonne surmontée d'un "I" représente la fraction éluée entre 0,12 et 0,2 M de NaCl sur DEAE-Sépharose, les quatrième, cinquième et sixième colonnes surmontée d'un "II" représentent les trois élutions successives effectuées sur colonne Con A-Sépharose et sont surnotées respectivement par les numéros d'ordre 1, 2 et 3, dans l'ordre de la description qui précède. Les deux dernières colonnes correspondent aux deux bandes gliotoxiques séparées et extraites d'un gel SDS-PAGE préparatif.

De ces résultats, il ressort que, comme dans l'analyse effectuée sur fluides biologiques de SEP sur colonne superose 12 et en SDS-PAGE unidimensionnelle à partir d'éluats de filtres utilisés pour la filtration in vivo du LCR de SEP, l'activité gliotoxique est retrouvée associée à des protéines de poids moléculaire apparent d'environ 17 KD et 21 KD, avec une concentration nettement moindre pour la molécule à 21 KD. Cependant, et contrairement aux observations effectuées précédemment, des échantillons témoins non-gliotoxiques passés sur colonne Con A-Sépharose dans les mêmes conditions ont présenté ces bandes à 17 KD et 21 KD sur gel SDS-PAGE dans la fraction éluée en tampon glycine HCl. L'évaluation dans les conditions décrites ci-dessus, de l'activité gliotoxique de ces échantillons témoins ne révèle pourtant aucun effet cytotoxique significatif à aucune étape de purification et pas plus au niveau des bandes protéiques à environ 17 KD et environ 21 KD. De plus, l'élution "à blanc" de colonne Con A-Sépharose avec le tampon glycine HCl pH 3 précité, permet de visualiser les mêmes bandes non-gliotoxiques. L'éventualité d'un décrochage de sous-unités ou de fragments protéiques de Con A à partir du support de chromatographie par le tampon glycine pH 3 a été vérifiée. Cependant, afin de vérifier la réalité d'un co-migration d'une molécule originale portant l'activité gliotoxique retrouvée dans les bandes d'environ 17 et 21 KD provenant d'échantillons de SEP élués sur Con A-Sépharose dans les conditions précitées, les inventeurs ont procédé à une digestion, dans les conditions adéquates, par la protéinase K de la fraction issue d'un échantillon gliotoxique et d'un échantillon témoin non-gliotoxique éluée dans les mêmes conditions sur Con A-Sépharose par le tampon glycine HCl. Les deux produits de digestion ont ensuite été élués en parallèle sur colonne FPLC superose 12 dans les conditions précédemment citées. Ces analyses ont permis de mettre en évidence que dans dans l'éluat provenant de l'échantillon "SEP" un pic protéique non-digéré, associé à une activité gliotoxique et exempt d'activité protéasique était toujours présent, alors que tout le matériel protéique était dégradé dans les fractions isssues d'échantillons témoins non-gliotoxiques contenant préalablement des protéines en 17 et 21 KD.

Un exemple de cette analyse est présenté dans les figures 12 et 13. Dans cet exemple, un mélange de surnageants de monocytes / macrophages de SEP présentant une activité gliotoxique significative et contenant 3 g de protéines et un échantillon équivalent provenant d'une culture témoin sans activité gliotoxique significative ont été passés en parallèle sur colonne FPLC DEAE-Sépharose, les fractions éluées entre 0,12 et 0,2 M NaCl récupèrées et l'équivalent de 2 mg de protéines pour chaque échantillon passés sur colonne FPLC Con A-Sépharose. Les fractions éluées dans le tampon glycine HCl 50 mM pH 3 avec 1 mM de Ca$^{++}$ et 1 mM de Mn$^{++}$, ont été d'abord remises en solution dans un tampon adéquat. Pour ce faire, les échantillons ont été lyophilisés, resuspendus dans 2,5 ml d'eau distillée stérile, déposés sur une colonne de chromatographie de type NAP-25 (Pharmacia) préalablement lavée dans 10 volumes de tampon 20 mM Tris-HCl, pH 8.0, 1 mM Ca$^{++}$, 0.1% SDS, puis élués avec le même tampon et utilisés comme tels pour l'incubation avec la protéinase K. L'enzyme utilisée est immobilisée (Proteinase K-Acrylic beads, Sigma ref. P0803) et utilisée sous un rapport de 20 mU / 50-100 µg de protéine, dans le tampon 20 mM Tris-HCl à pH 8,0 avecl mM Ca$^{++}$ et 0,1% SDS. Les échantillons à digèrer ont été incubés au moins 16 heures à 37°C. Les surnageants récupèrés après centrifugation et sédimentation des billes couplées à la protéinase K ont été ensuite passés sur colonne FPLC superose 12 dans les conditions décrites précédemment et éluées avec un tampon 50mM Tris-HCl pH 6,8 contenant 8 M d'urée afin, notamment, de dissocier toute protéine multimérique. La figure 12 représente l'élution en superose 12 de l'échantillon gliotoxique provenant de SEP après traitement par la protéinase K. La figure 13 représente l'élution en superose 12 de l'échantillon non gliotoxique provenant de témoins non-atteints de SEP après traitement par la protéinase K. Les courbes en trait noir continu représentent l'absorption à 280 nm de l'éluat, avec une mesure de densité optique de sensibilité R = 0,02, indiquant la concentration relative en protéine. Le point de la courbe correspondant à l'élution de protéines globulaires d'environ 17 KD est indiqué par une flèche. Un pic protéique d'environ 17 KD est visible seulement dans la figure 12, et est associé à une activité gliotoxique évaluée à 75% de cytotoxicité sur une dilution de l'échantillon au 1/10, selon le test biologique selon l'invention, après 72 h d'incubation et quantification de la mort cellulaire relative par le L/D test. Par ailleurs, afin de détecter toute trace de protéinase K contaminante, un test d'activité protéasique sur azocoll (Sigma ref. A9409) a été effectué et n'a pas permis de déceler de protéase contaminante à ce niveau. Par contre, un pic similaire d'absorption à 280 nm est décelé dans les deux échantillons en dessous de 5 KD, et correspond aux produits de dégradation par la protéinase K des protéines digestibles.

Les résultats présentés dans l'exemple illustré par les figures 12 et 13, montrent qu'il existe bien un facteur gliotoxique spécifique, présent de manière originale dans les fluides biologiques issus de patients atteints de SEP, associé à une ou deux molécule(s) polypeptidiques dont au moins une (ou des) région(s) de 17 KD est (sont) à indigestible(s) par la protéinase K. Cependant, le mode de purification utilisant des colonnes Con A-Sépharose, s'il a permis de mettre en évidence une trés forte affinité du facteur gliotoxique pour la concanavaline A, ne permet pas d'obtenir des échantillons adéquats pour une analyse peptidique ultérieure du fait d'une contamination par des composants de la concanavaline A provenant des colonnes dans les conditions d'élution requises pour éluer ledit facteur gliotoxique. Dans ces conditions, il semble qu'un protocole de purification associant successivement une colonne DEAE-Sépharose et une colonne superose 12, soit le mieux adapté à une stratégie de purification de molécules portant l'activité gliotoxique spécifique caractérisée dans la présente invention.

Un exemple d'analyse du rendement de purification par différentes colonnes FPLC est présenté dans le tableau 3.

Dans cet exemple, la cytotoxicité est exprimée en quantité de protéines (mg) nécessaire pour avoir 50% de mort cellulaire, d'après une quantification par un dosage colorimétrique au méthyltétrazolium après incubation à 37°C pendant 72 h, de l'échantillon à tester dilué au 1/10 dans le milieu de culture. Les fractions testées sont, pour chaque type de colonne, celles qui sont éluées dans les conditions préalablement décrites pour contenir l'activité gliotoxique. Le rendement est calculé selon la formule suivante :

$$\text{rendement } \% = \frac{\text{protéines totales (avp) / cytotoxicité (avp)}}{\text{protéines totales (app) / cytotoxicité (app)}} * 100$$

avp: avant purification; app: après purification.

Dans cet exemple, il apparait que les colonnes protéine A-sépaharose pourraient aussi être utilisées à bon escient, préalablement à une autre méthode de séparation excluant les colonnes Con A-Sépharose, dans une stratégie de purification du facteur gliotoxique.

Enfin, après avoir étudié différentes techniques de purification et de préparation de fractions moléculaires associées à l'activité gliotoxique mise en évidence dans les fluides biologiques issus de patients atteints de SEP, les inventeurs ont vérifié dans l'étude présidant à la présente invention que la purification, même partielle, de molécules constituant le fondement moléculaire de l'activité gliotoxique présente un effet dose-réponse encore plus clairement définissable que sur les fluides bruts précédemment testés, ceci, notamment, afin de vérifier la réalité bio-pharmacologique de la purification dudit facteur gliotoxique.

Les deux exemples qui suivent illustrent la réalité d'une purification d'une activité bio-pharmacologique parallèlement à la purification moléculaire effectuée.

Dans l'exemple illustré par la figure annexée N°14, dont les courbes ont été tracées d'après les données présentées dans le tableau annexé N°4, on a réalisé une série de dilutions dans du tampon PBS avec un surnageant de culture de monocyte/macrophage de patient atteint de SEP (SEP 1) et lesdites dilutions ont été incubées 72 heures avec une dilution finale allant du 1/20ème au 1/5000ème dans le milieu de culture de puits comprenant une monocouche de cellules astrocytaires provenant de la lignée astrocytaire décrite précédemment. Une fraction du même surnageant de culture de monocyte de SEP obtenue après purification de la fraction gliotoxique par passage sur colonne Con A-Sépharose, a été diluée et incubée en parallèle, selon le même protocole. Les cellules astrocytaires ont été incubées juste avant l'introduction des dilutions gliotoxiques, avec du Chrome 51 et lavées afin d'éliminer les isotopes radioactifs non incorporés dans les cellules vivantes. Après 72 heures d'incubation avec les deux gammes d'échantillons dilués, la radioactivité relarguée dans le surnageant est mesurée avec un compteur gamma et comparée à celle mesurée dans les cellules restant en culture au fond des puits. Le pourcentage de cytotoxicité, ainsi qu'expliqué précédemment, représente la proportion de radioactivité relarguée dans le surnageant par les cellules mortes. On peut ainsi voir sur la figure 14, qu'il existe un effet dose-réponse marqué par une diminution progressive de la cytotoxicité mesurée en fonction des dilutions croissantes de l'échantillon. Il faut cependant remarquer que la pente de la courbe est nettement accrue, sur les mêmes écarts de dilution, avec le facteur purifié. Ceci confirme la réalité de la purification et de la concentration moléculaire du facteur associé à l'activité biologique mesurée par notre test de gliotoxicité.

Dans l'exemple illustré par la figure 15 annexée dont les courbes ont été tracées d'après les données présentées dans le tableau 5 annexé, on a réalisé une série de dilutions dans du tampon PBS avec un surnageant de culture de monocyte/macrophage de patient atteint de SEP (SEP 1) et lesdites dilutions ont été incubées 72 heures avec une dilution finale allant du 1/20ème au 1/5000ème dans le milieu de culture de puits comprenant une monocouche de cellules astrocytaires provenant de la lignée astrocytaire décrite précédemment. Une fraction du même surnageant de culture de monocyte de SEP obtenue après purification de la fraction gliotoxique par passage sur colonne Con A-Sépharose, a été diluée et incubée en parallèle, selon le même protocole. Après 72 heures d'incubation avec les deux gammes d'échantillons dilués, les cellules restant viables dans les puits de culture sont détectées par le test au méthyltétrazolium (MTT) décrit précédemment et le produit coloré généré par les enzymes mitochondriales fonctionnelles de ces cellules est dosé par une mesure de la densité optique du surnageant entre 570 et 630 nm. La densité optique, ainsi qu'expliqué précédemment, représente la quantité de cellules vivantes restant dans chaque puits préalablement ensemencé avec le même nombre de cellules maintenues en monocouche confluente au fond du puits de culture, dans un milieu de survie. On peut ainsi voir sur la figure 15, qu'il existe bien un effet dose-réponse marqué par une augmentation progressive de la survie cellulaire mesurée en fonction des dilutions croissantes de l'échantillon. Il faut cependant remarquer que la pente de la courbe est nettement accrue, sur les mêmes écarts de dilution, avec le facteur purifié. Ceci confirme encore, par cette technique de quantification des cellules vivantes, la réalité de la purification et de la concentration moléculaire du facteur associé à l'activité biologique mesurée par notre test de gliotoxicité.

Dans les deux derniers exemples illustrés par les figures 14 et 15, deux techniques de dosages mesurant des paramètres opposés tels que la survie cellulaire et la mortalité cellulaire relative, procurent des résultats tout à fait convergeants et, tout en permettant de démontrer la réalité d'une purification de l'activité gliotoxique détectée en même temps que du facteur gliotoxique, montrent clairement la résolutivité et la fiabilité du test biologique faisant, ainsi que le facteur gliotoxique caractérisé par les travaux des auteurs, objet de la présente invention.

Ainsi, sur la base de la découverte d'une activité cytotoxique pouvant être mise en évidence in vivo chez des personnes atteintes de SEP et ciblant préférentiellement les cellules gliales, un procédé de détection et de quantification de l'activité cytotoxique et, plus particulièrement, gliotoxique associée à ce facteur a été inventé, mis au point et validé dans différentes conditions d'utilisation. De plus, ledit procédé a permis d'étudier et de caractériser le fondement moléculaire de cette activité gliotoxique, sous la forme de fractions protéiques présentant deux poids moléculaires apparents d'environ 17 KD et d'environ 21 KD, dans les liquides biologiques provenant, notamment, de patients atteints de SEP. Le fait qu'il existe une partie indigestible par la protéinase K dans des conditions non-dénaturantes, qui est retrouvée associée à l'activité gliotoxique à 17 KD par élution sur colonne FPLC superose 12 suggère éventuellement qu'un peptide additionnel digestible (propeptide par exemple) différencie la forme de 21 KD de celle de 17 KD. Ces deux facteurs apparamment protéiques d'environ 17 et d'environ 21 KD sont vraisemblablement globulaires, sans ponts disulfures associant des chaînes peptidiques indépendantes, plutôt hydrophiles, chargés négativement à pH neutre, et apparemment glycosylés sans pour autant que l'incubation en présence de N-glycosidase F et de neuraminidase n'abolisse leur activité gliotoxique. De plus, ils présentent une très haute affinité pour au moins une lectine, la concanavaline A, ou Con A. Ces facteurs protéiques ont une activité biologique qui résiste à l'incubation à 56°C pendant une demi-heure et qui disparait après chauffage à 100°C pendant 15 minutes. Cette activité biologique, autant que la forme à 17 KD résistent à l'action des protéases telles que la pronase, la trypsine et la protéinase K.

La description détaillée, précédemment exposée, a finalement permis de mettre en évidence un facteur gliotoxique possédant les caractéristiques suivantes prises indépendamment :

- il possède une activité cytotoxique sur les cellules gliales,
- cette activité cytotoxique sur les cellules gliales est associée à au moins une glycoprotéine globulaire,
- son activité est liée à au moins deux fractions protéiques, associées ou non, ayant un poids moléculaire apparent respectivement de 17 KD et de 21 KD, chacune de ces fractions possédant une activité gliotoxique, la fraction de 17 KD étant indigestible par la pronase, ou la trypsine ou la protéinase K, et chacune des deux fractions présentant une forte affinité pour les lectines telles que la concanavaline-A,
- l'activité gliotoxique du facteur présent dans un échantillon persiste après un traitement thermique de l'échantillon à +56°C de 30 minutes, ou après congélation de l'échantillon à -80°C puis décongélation jusqu'à +37°C,
- le facteur est soluble dans l'eau et non particulaire,
- l'activité gliotoxique du facteur présent dans un échantillon persiste après traitement de l'échantillon avec la trypsine, ou la pronase ou la protéinase K, ou un mélange de N-glycosidase F et de neuraminidase, dans des conditions non dénaturantes,
- le facteur caractérisé par électrophorèse sur gel de SDS-polyacrylamide à une dimension peut présenter deux bandes, de 17 KD et 21 KD,
- le facteur gliotoxique est retenu avec une forte affinité sur des supports de lectine,
- le facteur est élué à pH 7,5 dans un tampon de 100mM NaCl sur résine DEAE,
- il n'est pas retenu par la protéine A couplée à un support, donc il est différentiable des IgG,
- il est retrouvé dans les surnageants de cultures de monocytes/macrophages de SEP, les LCR et sérums de SEP,
- il provoque un effet cytotoxique quantifiable sur les cellules astrocytaires en culture et caractérisable par un effet précoce de désorganisation du réseau de filaments intermédiaires suivie habituellement par la mort cellulaire.

TABLEAU 1:

| EXEMPLE D'APPLICATION DU TEST BIOLOGIQUE DE GLIOTOXICITE | | | | |
|---|---|---|---|---|
| Filtration des liquides céphalo-rachidiens de patients atteints de SEP avec les filtres PALL | | | | |
| Pourcentage de cellules mortes (L/D test) | | | | |
| N* Patient | stade clinique | Avant filtration | Après filtration | Significativité (t-test) |
| 1 | Poussée | 14.4±1.7 | 1.4±1.7 | $2.49^{-10}$ |
| 2 | Poussée | 35.6±4.55 | 9.7±3.6 | $1.38^{-11}$ |
| 3 | Poussée | 20.3±3.59 | 3.2±2.2 | $1.93^{-6}$ |
| 4 | Poussée | 29.9±3.28 | 9.7±2.0 | $4.13^{-9}$ |
| 5 | Poussée | 45.0±3.46 | 4.2±2.7 | $4.16$-9 |
| 6 | Chronique | 20.8±4.13 | 19.6±2.41 | $5.15^{-1}$ NS |
| 7 | Chronique | 15.5±2.55 | 0.1±0.32 | $9.62^{-9}$ |
| 8 | Clinprobable | 24.1±3.67 | 0.6±0.84 | $5.55^{-9}$ |
| 9 | Poussée. | 30.4±4.97 | 4.1±2.38 | $1.46^{-8}$ |
| 10 | Chronique | 24.3±2.31 | 0.6±0.7 | $3.87^{-9}$ |

TABLEAU 1: (suite)

| Pourcentage de cellules mortes (L/D test) | | | | |
|---|---|---|---|---|
| N* Patient | stade clinique | Avant filtration | Après filtration | Significativité (t-test) |
| 11 | Chronique | 16.0±2.49 | 3.3±2.91 | $9.00^{-9}$ |
| 12 | Poussée | 34.9±3.75 | 0.3±0.68 | $4.20^{-9}$ |
| 13 | Poussée | 10.5±2.55 | 0 | $3.86^{-7}$ |
| 14 | Poussée | 0.6±0.84 | 0 | $5.10^{-2}$ NS |
| 15 | Chronique | 19.7±2.21 | 0.4±0.97 | $3.40^{-8}$ |
| 16 | Chronique stable | 34.5±1.72 | 4.8±2.1 | $3.72^{-9}$ |
| 17 | Chronique | 23.7±2.44 | 0 | $4.66^{-5}$ |
| 18 | Poussée | 37.9±1.88 | 2.77±1.55 | $3.98^{-8}$ |
| 19 | Poussée | 33.7±2.76 | 1.34±1.98 | $8.76^{-6}$ |
| 20 | Poussée | 29.5±2.98 | 2.76±2.21 | $7.45^{-5}$ |
| 21 | Chronique | 24.8±3.76 | 3.21±1.55 | $2.66^{-7}$ |
| 22 | Chronique | 24.9±2.88 | 2.54±2.12 | $3.11^{-8}$ |

* Moyenne des cellules dans 5 champs d'examination microscopiques choisis au hasard dans 2 puits dupliqués. Les cultures contrôles montrent une mort cellulaire de fréquence non significative [+] test "t" de student. NS=non significatif

TABLEAU 2:

| CYTOTOXICITE DU LCR DE PATIENTS ATTEINTS DE SEP OU D'AUTRE MALADIE NEUROLOGIQUE DETECTEE A L'AIDE DU TEST BIOLOGIQUE SUR LIGNEE D'ASTROCYTES IMMORTALISES ET QUANTIFIEE PAR LA METHODE COLORIMETRIQUE AU MTT | | |
|---|---|---|
| PATIENT n* | | CYTOTOXICITE (% cellules mortes) |
| SEP | FORME EVOLUTIVE | |
| 1 | Rémittante en poussée | 48.8±4.5 |
| 2 | Rémittante en poussée | 54.6±3.9 |
| 3 | Rémittante en poussée | 44.0±6.5 |
| 4 | Rémittante en poussée | 39.8±3.8 |
| 5 | Rémittante en poussée | 52.5±2.9 |
| 6 | Rémittante en poussée | 68.5±7.7 |
| 13 | Chronique | 7.2±2.2 |
| 14 | Chronique | 8.2±1.9 |
| 15 | Chronique | 4.1±3.0 |
| 16 | Chronique | 0 |
| 17 | Chronique | 5.4±1.7 |
| 18 | Chronique | 6.8±0.8 |
| HPN | (TEMOINS NON SEP) | |
| 19 | | 0 |
| 20 | | 0 |
| 21 | | 0 |
| 22 | | 0 |

TABLEAU 2:   (suite)

| CYTOTOXICITE DU LCR DE PATIENTS ATTEINTS DE SEP OU D'AUTRE MALADIE NEUROLOGIQUE DETECTEE A L'AIDE DU TEST BIOLOGIQUE SUR LIGNEE D'ASTROCYTES IMMORTALISES ET QUANTIFIEE PAR LA METHODE COLORIMETRIQUE AU MTT | | |
|---|---|---|
| HPN | (TEMOINS NON SEP) | |
| 23 | | 0 |
| 24 | | 0 |

LCR: Liquide Céphalo-rachidien: MTT: méthyltétrazol ium; SEP: Sclérose en plaques; HPN: Hydrocéphalie à pression normale.

La cytotoxicité a été mesurée avec la technique colorimétrique utilisant le MTT après 96 h d'incubation. Les échantillons de LCR ont été dilués au 1:20 dans le milieu de culture utilisé pour les astrocytes immortalisés. Chaque resultat représente la moyenne de deux expériences séparées et représentant chacune une série de 5 puits indépendants, soit, finalement, une moyenne de 10 valeurs par LCR testé.

Les échantillonsde LCR provenant de patients atteints de forme rémittente de SEP. on été prélevés eu moment de poussées cliniques.

La différence des moyennes de cytotoxicité dans les deux sous-populations de SEP, rémittantes versus chroniques, est statistiquement significative ($p < 0.0001$. Mann-Withney U test), la différence entre les résultats des LCR de SEP et de témoins atteints d'HPN est elle-même statistiquement significative ($p < 0.0001$. Mann-Withney U test) .

TABLEAU 3:

| EXEMPLE DE RENDEMENT DE PURIFICATION DU FACTEUR GLIOTOXIQUE A PARTIR DE SURNAGEANTS DE CULTURES DE MONOCYTES DE MALADES SEP | | |
|---|---|---|
| **surnageant** | | |
| | volume (ml) | 10,00 |
| | protéines (mg) | 42,00 |
| | purification | 1,0 |
| | cytotoxicité (mg) | 1,2 |
| | rendement (%) | 100 % |
| **protéine A-sépharose** | | |
| | volume (ml) | 8,00 |
| | protéines (mg) | 35,30 |
| | purification | 1,1 |
| | cytotoxicité (mg) | 1,08 |
| | rendement (%) | 93 % |
| **concanavaline A-sépharose + NAP-25** | | |
| | volume (ml) | 3,50 |
| | protéines (mg) | 0,32 |
| | purification | 100 |
| | cytotoxicité (mg) | 0,012 |
| | rendement (%) | 76 % |

TABLEAU 4:

| EFFET DOSE-REPONSE: QUANTIFICATION DE L'ACTIVITE GLIOTOXIQUE PAR LE TEST DE RELARGAGE DE 51-Cr | | | | | | |
|---|---|---|---|---|---|---|
| ECHANTILLON | DILUTION | AVANT PURIFICATION % de cytotoxicité (en trois mesures) | | | APRES PURIFICATION % de cytotoxicité (en trois mesures) | | |
| Milieu de culture | 1/1 | 7.200 | 10.000 | 7.600 | 11.400 | 8.200 | 7.400 |

TABLEAU 4:   (suite)

| EFFET DOSE-REPONSE: QUANTIFICATION DE L'ACTIVITE GLIOTOXIQUE PAR LE TEST DE RELARGAGE DE 51-Cr | | | | | | | |
|---|---|---|---|---|---|---|---|
| ECHANTILLON | DILUTION | AVANT PURIFICATION % de cytotoxicité (en trois mesures) | | | APRES PURIFICATION % de cytotoxicité (en trois mesures) | | |
| Patient contrôle | 1/20 | 11.400 | 10.800 | 10.200 | 7.100 | 6.600 | 8.200 |
| SEP 1 | 1/20 | 47.400 | 42.800 | 39.900 | 94.400 | 90.800 | 92.200 |
| SEP 1 | 1/200 | 39.000 | 40.400 | 43.100 | 78.800 | 74.200 | 79.400 |
| SEP 1 | 1/1000 | 36.200 | 32.400 | 33.300 | 72.100 | 70.400 | 70.000 |
| SEP 1 | 1/5000 | 28.100 | 26.600 | 26.400 | 51.200 | 48.400 | 46.200 |

TABLEAU 5:

| EFFET DOSE-REPONSE: QUANTIFICATION DE L'ACTIVITE GLIOTOXIQUE PAR LE TEST COLORIMETRIQUE AU MTT | | | | | | | |
|---|---|---|---|---|---|---|---|
| ECHANTILLON | DILUTION | AVANT PURIFICATION D.0. 570-630 nm (en trois mesures) | | | APRES PURIFICATION D.0. 570-630 nm (en trois mesures) | | |
| Milieu de culture | 1/1 | 1.904 | 1.870 | 1.884 | 1.922 | 1.910 | 1.934 |
| Patient contrôle | 1/20 | 1.912 | 1.892 | 1.897 | 1.882 | 1.864 | 1.890 |
| SEP 1 | 1/20 | 1.604 | 1.572 | 1.608 | 0.948 | 0.990 | 0.960 |
| SEP 1 | 1/200 | 1.812 | 1.794 | 1.800 | 1.310 | 1.302 | 1.322 |
| SEP 1 | 1/1000 | 1.890 | 1.914 | 1.888 | 1.760 | 1.784 | 1.762 |
| SEP 1 | 1/5000 | 1.902 | 1.896 | 1.890 | 1.890 | 1.824 | 1.818 |

**BIBLIOGRAPHIE**

(1) Prineas J.W., The neuropathology of multiple sclerosis dans "Handbook of Clinical Neurology : Demyelinating Diseases", volume 3 n°47, Koetsier J.C. éditeur, 213-257, Elsevier, Amsterdam 1985.
(2) Prineas J.W., Barnard R.O., Kwon E.E., Sharer L.R. et Cho E.S., Multiple sclerosis: remyelination of nascent lesions. Ann. Neurol., 1993; 33, 137-151.
(3) Boyle E.A. et McGeer P.L., Cellular immune response in multiple sclerosis plaques, American Journal of Pathology, 1993; 137, 575-584.
(4) Charcot J.M., Histologie de la sclérose en plaques, Gaz. Hop. (Paris), 1868; 41, 554-566.
(5) Hauw J.J. et Escourolle R., Aspects anatomopathologiques de la sclérose en plaques, dans "La sclérose en plaques", Rascol A., Bés A. et Guiraud-Chaumeil B., 9-47. Masson, Paris, 1980.
(6) Poirier J., Fleury J., et Ghérardi R., La barrière hémato-encéphalique, Données morphologiques, La Revue de Médecine Interne, 1983; 4, 131-144.
(7) Netsky M.G., et Shuangshoti S., The choroid plexus in health and disease, University Press of Virginia, 1975.
(8) Gonzales-Scarano F., Grossman R.I., Galetta S. Atlas S.W. et Silberberg D.H., Multiple slerosis disease activity correlates with gadolinium enhancement magnetic resonance imaging, Ann. Neurol., 1987; 21, 300-306.
(9) Rapport S.I., Blood-brain barrier in physiology and medicine, Raven Press. 1976.
(10) Kent T.A. et McKendall R.R., Cerebral blood flow, cerebral metabolism and blood-brain barrier, In, McKendall R.R. Ed., Handbook of Clinical Neurology, Vol. 12, n°56: Viral disease, Elsevier, Amsterdam, 1989.
(11) Prineas J.W. et Wright R.G., Macrophages, lymphocytes, and plasma cells in the perivascular compartment in chronic multiple sclerosis, Laboratory Investigation, 1978; 38, 409-421.
(12) Bergamini L, et Durell L., Multiple sclerosis, I, The immune pathogenetic hypothesis, Riv. Neurol., 1989; 59, 176-90.
(13) Calder V, Owen S, Watson C, Feldmann M, et Davidson A., MS : a localized immune disease of central nervous system, Immunol Today, 1989; 10, 99-103.

**(14)** Jervis G.A., et Koprowski H., Chronic experimental allergic encephalomyelitis, J. Neuropathol. Exp. Neurol., 1948; 7, 309-320.

**(15)** Prineas JW., Pathology of early lesion in multiple sclerosis, Human pathology, 1975; 6, 23-7.

**(16)** Escourolle R., Hauw J.J. et Lyon-Caen O., Principales données morphologiques, approches physiopathologiques et étiologiques de la sclérose en plaques, La Revue du Praticien (Paris), 1980; 30, 2047-2053.

**(17)** Mc Donald W.I., The mystery of the origin of multiple sclerosis, J. Neurol. Neurosurg. Psych., 1986; 49, 113-123.

**(18)** Carp R.I., Warner H.B. et Merz G.S., Viral etiology of multiple sclerosis., Prog. Med. Virol., 1978; 24, 158-177.

**(19)** Marie P., Sclérose en plaques et maladies infectieuses, Le progrès médical, 1884; 12, 287-289.

**(20)** Gay D, Dick G, et Upson G., Multiple sclerosis caused by an oral spirochete?, Lancet ; 1986, 2, 815-9.

**(21)** De Keyser J. Autoimmunity in multiple sclerosis. Neurology, 1988 Mar, 38, 371-4.

**(22)** Juntunen J, Kinnunen E, Anti-Poika M, Koskenvuo M. Multiple sclerosis and occupational exposure to chemicals : a co-twin control study of a nationwide series of twins, Br. J. Int. Med., 1989; 417-9.

**(23)** Ebers G.C, Bulman D., The geographic distribution of MS reflects genetic susceptibilty, Neurology, 1986; 36, S1-108.

**(24)** Haegert D.G., Michaud M., Schwab C., Tansey C., Secary F., Francis G., HLA-DR beta, -DQ alpha and -DQ beta restriction fragment length polymorphisms in multiple sclerosis, J. Neurosci. Res., 1989; 23, 46-54.

**(25)** Waksman B.H., Mechanisms in Multiple Sclerosis, Nature, 1985; 318, 104-105.

**(26)** Acha-Orbea H. et Palmer E., Mls - a retrovirus exploits the immune system, Immunology Today 1991; 12, 356-361.

**(27)** Cole B.C et Atkin C.L., The mycoplasma arthritidis T-cell mitogen, MAM: a model superantigen, Immunology Today 1991; 12, 271-276.

**(28)** Rudge P., Does a retrovirally encoded superantigen cause multiple sclerosis?, Journal of Neurology, Neurosurgery and Psychiatry, 1991; 54, 853-855.

**(29)** Woodland D.L., Happ M.P., Gollob K.J. et Palmer E., An endogenous retrovirus mediating deletion of aβ T cells?, Nature (London), 1991; 349, 529-530.

**(30)** Traugott U., Multiple sclerosis: relevance of class I and class II MHC-expressing cells to lesion development, Journal of Neuroimmunology, 1987; 16, 283-302.

**(31)** Williams G.T. et Smith C.A., Molecular regulation of apoptosis: genetic controls on cell death, Cell, 1993; 74, 777-779.

**(32)** Levine B., Huang Q., Isaacs J.T., Reed J.C., Griffin D.E. et Hardwick J.M., Conversion of lytic to persistent alphavirus infection by the bcl-2 cellular oncogene, Nature, 1993; 361, 739-742.

**(33)** Newell M.K., VanderWall J., Beard K.S. et Freed J.H., Ligation of major histocompatibility complex class II molecules mediates apoptotic cell death in resting B lymphocytes, P.N.A.S., 1993; 90, 10459-10463.

**(34)** Selmaj. K.W. et Raine C.S., Tumor necrosis factor mediates myelin and oligodendrocyte damage in vitro, Ann. Neurol., 1988; 23, 339-346.

**(35)** Barna B.P., Estes M.L, Jacobs B.S., Hudson S. et Ransohoff R.M., Human astrocytes proliferate in response to tumor necrosis factor alpha, J. Neuroimmunol., 1990; 30, 239-243

**(36)** Robbins D.S., Shirazi Y., Drysdale B.E., Lieberman A., Shin H.S. et Shin M.L., Production of cytotoxic factor for oligodendrocytes by stimulated astrocytes, The Journal of Immunology 1987; 139, 2593-2597.

**(37)** Beck J., Rondot P., Catinot L., Falcoff E., Kirchner H. et Wietzerbin J., Increased production of interferon gamma and tumor necrosis factor precedes clinical manifestation in multiple sclerosis: do cytokine trigger off exacerbations ?, Acta Neurol. Scand., 1988; 78, 318-323.

**(38)** Kaufmann S.H.E., Heat Shock Proteins and Immune Response, Current Topics in Microbiology and Immunology, vol. 167, Springer-Verlag, Berlin, 1991.

**(39)** Wienfield J.B. et Jarjour W.N., Stress proteins, autoimmunity, and autoimmune disease, dans Heat Shock Proteins and Immune Response, Kaufmann S.H.E., Current Topics in Microbiology and Immunology, vol. 167, 161-189, Springer-Verlag, Berlin, 1991.

**(40)** Brocke S., Gaur A., Piercy C., Gautam A., Gijbels K., Fathman C.G. et Steinman L., Induction of relapsing paralysis in experimental autoimmune encephalomyelitis by bacterial superantigen, Nature, 1993; 365, 642-644.

**(41)** Birnbaum G., Kotilinek L. et Albrecht L., Spinal fluid lymphocytes from a subgroup of multiple sclerosis patients respond to mycobacterial antigens, Ann. Neurol. 1993; 34, 18-24.

**(42)** Ransohoff R.M. et Rudick R.A., Heat-shock proteins and autoimmunity: implications for multiple sclerosis, Annals of Neurology, 1993; 34, 5-7.

**(43)** Perron H. Geny C., Laurent A., et al., Leptomeningeal cell line from multiple sclerosis with reverse transcriptase activity and viral particles, Res. Virol., 1989; 140, 551-561.

**(44)** Perron H. , Geny C., Gratacap B., Laurent A., Mouriquand C., Pellat J., Perret J., et Seigneurin J.M., Isolation of an unknown retrovirus from CSF, blood and brain from patients with multiple sclerosis, in "Current concepts in multiple sclerosis", Wiethölter et al., pp.111-116. Elsevier, Amsterdam,1991.

**(45)** Perron H., Lalande B., Gratacap B., Laurent A., Genoulaz O., Geny C., Mallaret M., Schuller E., Stoebner P., et Seigneurin J.M., Isolation of retrovirus from patients with multiple sclerosis, Lancet, 1991; 337, 862-863.

**(46)** Dalgleish A.G., Fazakerley J.K. et Webb H.E., Do human T-lymphotropic viruses (HTLVs) and other enveloped viruses induce autoimmunity in multiple sclerosis?, Neuropath. Appl. Neurobiol., 1987; 13, 241-250.

**(47)** Birnbaum G., Kotilinek L. et Albrecht L., Spinal fluid lymphocytes from a subgroup of multiple sclerosis patients respond to mycobacterial antigens, Ann. Neurol., 1993; 34, 18-24.

**(48)** Davison A.N. et Sabri M.I., Biosynthesis of Myelin and Neurotoxic Factors in the Serum of Multiple Sclerosis Patients, Advances in Experimental Medicine, vol 100, 1978, New-York, US, 19-25.

**(49)** Poser C.M. et coll., New diagnostic criteria for multiple sclerosis : guidelines for research protocols, dans "The diagnosis of multiple sclerosis", Poser C.M., Paty D.W., Scheinberg L., Mac Donald W.I., Ebers G.C. pp.225-229, 1984, Thieme Stratton Inc., New-York.

**(50)** Galiana E., Borde I., Marin P., Rassoulzadegan M., Cuzin F., Gros F., Rouget P. et Evrard C., Establishment of permanent astroglial cell lines, able to differentiate in vitro, from transgenic mice carrying the polyoma virus large-T gene: an alternative approach to brain cell immortalization, Journal of Neuroscience Research, 1990; 26; 269-277.

**(51)** Mosmann T., Rapid colorimetric assay of cellular growth and survival: application to proliferation and cytotoxicity assays, J. Immunol. Meth., 1983; 65, 55-63.

**(52)** Wollinsky K.H., Hülser P.J., Mauch E., Mehrkens H.H. et Kornhuber H.H., Liquorpherese bei 10 Patienten mit Multipler sklerose dans verhandlungen der Deutschen Gesellschaft für Neurologie, Grundmann M et al, vol 7 (1992) Saarbrücken.

**(53)** Bradford M.M., A rapid sensitive method for the quantitation of microgram quantities of protein utilizing the principle of protein-dye binding, Anal. Biochem., 1976; 72, 248-254.

**(54)** Silberberg D. et coll., Tissue culture demyelination by normal human serum, Annals of Neurology, 1984: 15, 575-580.

## Revendications

**1.** Facteur gliotoxique **caractérisé en ce que**, après traitement d'un échantillon biologique le contenant, successivement, sur une colonne échangeuse d'ions puis sur une résine de séparation par exclusion, ledit facteur gliotoxique est constitué, majoritairement, par une fraction légère, centrée sur un poids moléculaire apparent d'environ 17 kD, et minoritairement, par une fraction lourde, centrée sur un poids moléculaire apparent d'environ 21 KD, au moins ladite fraction légère étant résistante, dans des conditions non dénaturantes, à l'action hydrolytique de la pronase, ou de la trypsine, ou de la protéinase K, et chacune des deux dites fractions présentant une forte affinité pour les lectines et notamment la concanavaline-A.

**2.** Facteur gliotoxique selon la revendication 1, **caractérisé en ce qu'**il possède une activité toxique vis-à-vis des cellules astrocytaires humaines ou animales, ayant pour effet, à la fois, une désorganisation cytomorphologique de leur réseau de filaments intermédiaires, une dégradation des protéines desdits filaments intermédiaires, et une mort cellulaire par apoptose.

**3.** Facteur selon la revendication 2, **caractérisé en ce que** son activité est associée à au moins une glycoprotéine globulaire.

**4.** Facteur gliotoxique selon l'une quelconque des revendications précédentes, **caractérisé en ce qu'**il est susceptible d'être obtenu à partir du procédé comprenant les étapes suivantes:

- on part d'un échantillon biologique prélevé par exemple sur un patient atteint de sclérose en plaques cliniquement active,
- on traite successivement ledit échantillon sur une résine échangeuse d'ions puis sur une colonne de séparation par exclusion, pour obtenir ledit facteur gliotoxique.

**5.** Procédé pour détecter et/ou suivre et/ou pronostiquer l'activité d'une pathologie telle que la sclérose en plaques, **caractérisé en ce qu'**on détecte, dans un échantillon biologique, la présence et/ou la quantité d'un facteur gliotoxique selon l'une quelconque des revendications précédentes.

**EP 0 667 354 B1**

**6.** Procédé de prétraitement d'un échantillon biologique contenant un facteur selon l'une quelconque des revendications 1 à 4, **caractérisé en ce qu'**on effectue un traitement dudit échantillon avec l'un au moins des traitements suivants:

- on met ledit échantillon en contact avec de la protéine A,
- on met ledit échantillon en contact avec une résine échangeuse d'ions,
- on met ledit échantillon en contact avec une lectine et notamment la concanavaline-A.

**7.** Procédé pour détecter et/ou quantifier, dans un échantillon biologique, l'activité toxique du facteur gliotoxique selon l'une quelconque des revendications 1 à 4, **caractérisé en ce qu'**on incube ledit échantillon biologique dans un milieu de culture approprié et contenant des astrocytes, notamment immortalisés, permettant leur culture, et en ce qu'on détecte et/ou quantifie les astrocytes morts et/ou les astrocytes vivants.

**8.** Procédé selon la revendication 7, **caractérisé en ce que**, pour détecter et/ou quantifier les astrocytes morts et/ou les astrocytes vivants, on utilise un dosage colorimétrique mettant en oeuvre respectivement la calcéine-AM et l'éthidium homodimère.

**9.** Procédé selon la revendication 7, **caractérisé en ce que**, pour détecter et/ou quantifier les astrocytes vivants, on utilise un dosage colorimétrique mettant en oeuvre le bromure de méthyltétrazolium.

**10.** Procédé selon la revendication 9, **caractérisé en ce que**, pour détecter et/ou quantifier les astrocytes morts, on utilise un dosage radioactif mettant en oeuvre le $^{51}$Cr.

**11.** Procédé pour détecter et/ou quantifier, dans un échantillon biologique, l'activité toxique d'un facteur gliotoxique selon l'une quelconque des revendications 1 à 4, **caractérisé en ce qu'**on incube ledit échantillon dans un milieu de culture approprié, contenant des astrocytes, notamment immortalisés, et permettant leur culture, et en ce qu'on détecte et/ou quantifie la fragmentation de l'ADN des astrocytes, et/ou la désorganisation cytomorphologique du réseau des filaments intermédiaires et/ou la dégradation des protéines desdits filaments intermédiaires.

**12.** Procédé pour détecter, dans un échantillon biologique, la présence d'un facteur selon l'une quelconque des revendications 1 à 4, **caractérisé en ce qu'**on réalise une étape de capture dudit facteur par une lectine et notamment la concanavaline-A, et/ou une étape de détection basée sur l'affinité dudit facteur pour unedite lectine.

**13.** Composition diagnostique, et/ou thérapeutique, et/ou prophylactique, **caractérisée en ce qu'**elle comprend un facteur tel que défini selon l'une quelconque des revendications 1 à 4.

**Patentansprüche**

**1.** Gliotoxischer Faktor, **dadurch gekennzeichnet, daß** nach sukzessiver Behandlung einer diesen enthaltenden biologischen Probe auf einer Ionenaustauschersäule und anschließend auf einem Ausschlußtrennharz der gliotoxische Faktor hauptsächlich durch eine leichte Fraktion, die um ein apparentes Molekulargewicht um etwa 17 kD zentriert ist, und zum kleinen Teil durch eine schwere Fraktion gebildet wird, die um ein apparentes Molekulargewicht von etwa 21 kD zentriert ist, wobei zumindest die leichte Fraktion unter nicht-denaturierenden Bedingungen gegenüber der hydrolytischen Aktivität der Pronase oder des Trypsins oder der Proteinase K resistent ist, und jede der beiden Fraktionen eine starke Affinität zu Lektinen und insbesondere zu Concanavalin-A zeigt.

**2.** Gliotoxischer Faktor nach Anspruch 1, **dadurch gekennzeichnet, daß** er eine gegenüber humanen oder tierischen astrocytären Zellen toxische Aktivität hat, was gleichzeitig eine cytomorphologische Desorganisation ihres Geflechts von Intermediärfilamenten, eine Degradation der Proteine der Intermediärfilamente und ein zelluläres Absterben durch Apoptose bewirkt.

**3.** Faktor nach Anspruch 2, **dadurch gekennzeichnet, daß** seine Aktivität mit zumindest einem globulären Glykoprotein assoziiert ist.

**4.** Gliotoxischer Faktor nach einem der vorherigen Ansprüche, **dadurch gekennzeichnet, daß** dieser ausgehend von einem Verfahren erhalten werden kann, das nachfolgende Schritte aufweist:

**25**

- es wird von einer biologischen Probe ausgegangen, die z.B. einem an klinisch aktiver multipler Sklerose erkrankten Patienten entnommen wurde, und

- die Probe wird sukzessive auf einem Ionenaustauscherharz und anschließend auf einer Ausschlußtrennsäule behandelt, um den gliotoxischen Faktor zu erhalten.

5. Verfahren, um die Aktivität einer Pathologie, wie z.B. der multiplen Sklerose, nachzuweisen und/oder zu verfolgen und/oder zu prognostizieren, **dadurch gekennzeichnet, daß** in einer biologischen Probe das Vorhandensein und/oder die Menge eines gliotoxischen Faktors nach einem der vorherigen Ansprüche detektiert wird.

6. Verfahren, um eine biologische Probe vorzubehandeln, die einen Faktor nach einem der Ansprüche 1 bis 4 enthält, **dadurch gekennzeichnet, daß** eine Behandlung der Probe mit zumindest einer der nachfolgenden Handlungen durchgeführt wird:

- In-Kontakt-Bringen der Probe mit Protein A,

- In-Kontakt-Bringen der Probe mit einem Ionenaustauscherharz,

- In-Kontakt-Bringen der Probe mit einem Lektin und insbesondere mit Concanavalin-A.

7. Verfahren, um in einer biologischen Probe die toxische Aktivität des gliotoxischen Faktors nach einem der Ansprüche 1 bis 4 nachzuweisen und/oder zu quantifizieren, **dadurch gekennzeichnet, daß** die biologische Probe in einem geeigneten Kulturmedium inkubiert wird, das Astrocyten, insbesondere immortalisierte Astrocyten, enthält, und das deren Kultivierung ermöglicht, und daß die abgestorbenen Astrocyten und/oder lebenden Astrocyten detektiert und/oder quantifiziert werden.

8. Verfahren nach Anspruch 7, **dadurch gekennzeichnet, daß** zum Detektieren und/oder Quantifizieren der abgestorbenen Astrocyten und/oder lebenden Astrocyten eine colorimetrische Bestimmungsmethode verwendet wird, bei der Calcein-AM beziehungsweise Ethidiumhomodimer eingesetzt wird.

9. Verfahren nach Anspruch 7, **dadurch gekennzeichnet, daß** zum Detektieren und/oder Quantifizieren der lebenden Astrocyten eine colorimetrische Bestimmungsmethode verwendet wird, bei der Methyltetrazoliumbromid eingesetzt wird.

10. Verfahren nach Anspruch 9, **dadurch gekennzeichnet, daß** zum Detektieren und/oder Quantifizieren der abgestorbenen Astrocyten eine radioaktive Bestimmungsmethode verwendet wird, bei der $^{51}$Cr eingesetzt wird.

11. Verfahren, um in einer biologischen Probe die toxische Aktivität eines gliotoxischen Faktors nach einem der Ansprüche 1 bis 4 zu detektieren und/oder zu quantifizieren, **dadurch gekennzeichnet, daß** die Probe in einem geeigneten Kulturmedium inkubiert wird, das Astrocyten, insbesondere immortalisierte Astrocyten, enthält, und das deren Kultivierung ermöglicht, und daß die Fragmentierung der DNA der Astrocyten und/oder die cytomorphologische Desorganisation des Geflechts der Intermediärfilamente und/oder die Degradation der Proteine der Intermediärfilamente detektiert und/oder quantifiziert werden.

12. Verfahren, um in einer biologischen Probe das Vorhandensein eines Faktors nach einem der Ansprüche 1 bis 4 zu detektieren, **dadurch gekennzeichnet, daß** ein Schritt zum Einfangen des Faktors durch ein Lektin und insbesondere Concanavaline-A, und/oder ein Detektionsschritt, der auf der Affinität des Faktors zu einem Lektin basiert, durchgeführt wird.

13. Diagnostische und/oder therapeutische und/oder prophylaktische Zusammensetzung, **dadurch gekennzeichnet, daß** sie einen nach einem der Ansprüche 1 bis 4 definierten Faktor enthält.

## Claims

1. Gliotoxic factor, **characterized in that**, after successive treatment of a biological sample containing said factor, on an ion exchange resin and then on a resin for separation by exclusion, said gliotoxic factor consists preponderantly of a light fraction centered around an apparent molecular weight of approximately 17 kD, and of a less abundant heavy fraction centered around an apparent molecular weight of approximately 21 kD, at least said light fraction being resistant, under nondenaturing conditions, to the hydrolytic action of pronase or trypsin or proteinase K, and each of the two said fractions displaying a strong affinity for lectins and in particular concanavalin A.

2. Gliotoxic factor according to claim 1, **characterized in that** it possesses toxic activity with respect to human or animal astrocytic cells, having the effect of a cytomorphological disorganization of their network of intermediate filaments, a degradation of the proteins of said intermediate filaments and cell death by apoptosis.

3. Factor according to claim 2, **characterized in that** its activity is associated with at least one globular glycoprotein.

4. Gliotoxic factor according to anyone of the preceding claims, **characterized in that** it is capable of being obtained using the method comprising the following steps:

   - the starting material is a biological sample taken, for example, from a patient suffering from clinically active multiple sclerosis,
   - said sample is treated successively on an ion exchange resin and then on a column for separation by exclusion, to obtain said gliotoxic factor.

5. Method for detecting and/or monitoring and/or predicting the activity of a pathology such as multiple sclerosis, **characterized in that** the presence and/or the amount of a gliotoxic factor according to any one of the preceding claims is/are detected in a biological sample.

6. Method of pretreatment of a biological sample containing a factor according to any one of claims 1 to 4, **characterized in that** a treatment of said sample with at least one of the following treatments is performed:

   - said sample is brought into contact with protein A,
   - said sample is brought into contact with an ion exchange resin,
   - said sample is brought into contact with a lectin and in particular concanavalin A.

7. Method for detecting and/or quantifying, in a biological sample, the toxic activity of the gliotoxic factor according to any one of claims 1 to 4, **characterized in that** the said biological sample is incubated in a suitable culture medium containing astrocytes, in particular immortalized astrocytes, enabling them to be cultured, and in that the dead astrocytes and/or the living astrocytes are detected and/or quantified.

8. Method according to claim 7, **characterized in that**, to detect and/or quantify the dead astrocytes and/or the living astrocytes, a colorimetric assay method employing calcein-AM and ethidium homodimer, respectively, is used.

9. Method according to claim 7, **characterized in that**, to detect and/or quantify the living astrocytes, a colorimetric assay method employing methyltetrazolium bromide is used.

10. Method according to claim 9, **characterized in that**, to detect and/or quantify the dead astrocytes, a radioactive assay method employing $^{51}$Cr is used.

11. Method for detecting and/or quantifying, in a biological sample, the toxic activity of a gliotoxic factor according to any one of claims 1 to 4, **characterized in that** said sample is incubated in a suitable culture medium containing astrocytes, in particular immortalized astrocytes, and enabling them to be cultured, and in that fragmentation of the DNA of the astrocytes and/or the cytomorphological disorganization of the network of intermediate filaments and/or the degradation of the proteins of said intermediate filaments is/are detected and/or quantified.

12. Method for detecting, in a biological sample, the presence of a factor according to any one of claims 1 to 4, **characterized in that** a step of capture of said factor by a lectin and in particular concanavalin A, and/or a detection step based on the affinity of said factor for a said lectin, is/are carried out.

13. Diagnostic and/or therapeutic and/or prophylactic composition, **characterized in that** it comprises a factor, according to any one of claims 1 to 4.

FIG 1

FIG 2

FIG 3

A

B

FIG 4

FIG 5

EP 0 667 354 B1

FIG6

FIG7

# FIG 8A

# FIG 8A

FIG 9

FIG 10A

FIG 10B

Ve ~ 16,8 kD

Ve ~ 21 kD

Ve ~ 110 kD

EP 0 667 354 B1

# FIG 11

I ... DEAE - Seph. [0.12 - 0.20 M NaCl]

II ... Con A - Seph.

III ... SDS - PAGE

FIG 12

Ve ≃ 17kD

(azocoll ⊖ )
72h, 37°C  75%tox

30  40  50  56  60 (16.8kD)  70

20  30  40  50  60  70  80  90

EP 0 667 354 B1

FIG 13

ConA + proteinase K

Ve < 5kD

Ve = 17kD     0% tox

EP 0 667 354 B1

## FIGURE 14

FIGURE 15